# EUROPEAN PATENT APPLICATION

(11) **EP 4 389 899 A1**
(43) Date of publication of application: **26.06.2024**
(21) Application number: 22386098.2
(22) Date of filing: 22.12.2022
(51) Int. Cl.: C12N 15/63

(54) **REGULATORY NUCLEIC ACID SEQUENCES**

(71) Applicant: Synpromics Ltd., Edinburgh EH25 9RG (GB); Asklepios Biopharmaceutical, Inc., Research Triangle Park, NC 27709 (US)
(72) Inventor: EVRIPIOTI, Antonia, Edinburgh EH25 9RG (GB)
(74) Representative: HGF

(57) **Abstract**

The present invention relates to regulatory nucleic acid sequences, in particular muscle-specific promoters, elements thereof, and other such nucleic acid sequences, that are capable of enhancing muscle-specific expression of genes. In particular, the invention relates to regulatory nucleic acid sequences capable of enhancing muscle-specific expression but having low liver-specific expression. The invention also relates to expression constructs, vectors and cells comprising such muscle-specific regulatory nucleic acid sequences, and to methods of their use. The regulatory nucleic acid sequences are of particular utility for gene therapy applications, but also find utility in other areas such as bioprocessing and biotechnology.

## Description

### Field of the Invention

The present invention relates to regulatory nucleic acid sequences, in particular muscle-specific promoters, elements thereof, and other such nucleic acid sequences, that are capable of enhancing muscle-specific expression of genes. In particular, the invention relates to regulatory nucleic acid sequences capable of enhancing muscle-specific expression but having low liver-specific expression. The invention also relates to expression constructs, vectors and cells comprising such regulatory nucleic acid sequences, and to methods of their use. The regulatory nucleic acid sequences are of particular utility for gene therapy applications, but also find utility in other areas such as bioprocessing and biotechnology.

### Background of the Invention

The following discussion is provided to aid the reader in understanding the disclosure and does not constitute any admission as to the contents or relevance of the prior art.

In many areas, including gene therapy, it is desirable to provide regulatory nucleic acid sequences that are capable of driving expression of a gene to produce a protein or nucleic acid expression product within a desired cell, tissue or organ.

Expression of therapeutic genes in muscle is attractive for gene therapy. Gene therapy in muscle has the potential to correct or augment the expression of various muscle proteins, such as dystrophin and sarcoglycans. This can be used to treat conditions such as muscular dystrophy, e.g. Duchenne muscular dystrophy (DMD). Muscle can also be used as a platform to express therapeutic protein for the treatment of other conditions.

Various vectors have been used to deliver genes to muscle cells, for example adenoviral, retroviral, lentiviral, and adeno-associated viral (AAV), as well as non-viral vectors such as plasmids. Adenoviral vectors have a comparatively large cloning capacity and can transduce some cells efficiently. However, they face significant challenges in view of the strong immune response they tend to elicit. Retroviral and lentiviral vectors stably integrate into the genome, which is associated with both benefits and disadvantages. Lentiviral vectors can transduce both dividing and non-dividing cells, but most conventional retroviral vectors can only transduce dividing cells, which limits their use in non-dividing muscle cells. Plasmid DNA can be used to transfer genes to muscle cell in vitro, but their potential utility in a clinical context is less clear.

AAV vectors are particularly attractive for gene therapy applications in muscle. AAV vectors display a natural tropism to muscle cells, can drive long-term expression of a therapeutic payload, and elicit a minimal immune response. Despite the ability of some gene therapy vectors to preferentially transduce muscle cells, off-target transduction does occur. Several Phase 1 and Phase 2 clinical trials using AAV serotypes 1, 2 and chimeric 2.5 have been reported for the treatment of Duchenne muscular dystrophy (DMD) and alpha- 1 antitrypsin deficiency (D. E. Bowles, S. WJ McPhee, C. Li, S. J. Gray, J. J. Samulski, A. S. Camp, J. Li, B. Wang, P. E. Monahan, J. E. Rabinowitz, J. C. Grieger, La. Govindasamy, M. Agbandje-McKenna, X. Xiao and R. J. Samulski, Molecular Therapy, 20, 443-455 (2012); M. L. Brantly, J. D. Chulay, L. Wang, C. Mueller, M. Humphries, L. T. Spencer, F. Rouhani, T. J. Conlon, R. Calcedo, M. R. Berts, C. Spencer, B. J. Byrne, J. M. Wilson, T. R. Flotte, Sustained transgene expression despite T lymphocyte responses in a clinical trial of rAAVI-AAT gene therapy. Proceedings of the National Academy of Sciences of the United States of America 106, 16363-16368 (2009); T. R. Flotte, M. L. Brantly, L. T. Spencer, B. J. Byrne, C. T. Spencer, D. J. Baker, M. Humphries, Phase I trial of intramuscular injection of a recombinant adeno-associated virus alpha 1 -antitrypsin (rAAV2-CB-hAAT) gene vector to AAT-deficient adults. Human gene therapy 15, 93-128 (2004); T. R. Flotte, B. C. Trapnell, M. Humphries, B. Carey, R. Calcedo, F. Rouhani, M. Campbell-Thompson, A. T. Yachnis, R. A. Sandhaus, N. G. McElvaney, C. Mueller, L. M. Messina, J. M. Wilson, M. Brantly, D. R. Knop, G. J. Ye, J. D. Chulay, Phase 2 clinical trial of a recombinant adeno-associated viral vector expressing alphal -antitrypsin: interim results. Human gene therapy 22, 1239-1247 (2011); C. Mueller, J. D. Chulay, B. C. Trapnell, M. Humphries, B. Carey, R. A. Sandhaus, N. G. McElvaney, L. Messina, Q. Tang, F. N. Rouhani, M. Campbell-Thompson, A. D. Fu, A. Yachnis, D. R. Knop, G. J. Ye, M. Brantly, R. Calcedo, S. Somanathan, L. P. Richman, R. H. Vonderheide, M. A. Hulme, T. M. Brusko, J. M. Wilson, T. R. Flotte, Human Treg responses allow sustained recombinant adeno-associated virus-mediated transgene expression. The Journal of clinical investigation 123, 5310-5318 (2013)).

One of the off-target effects persistently observed in clinical trials of AAV therapeutic products is liver toxicity. Recently, Audentes Therapeutics' phase 2 gene therapy trial for X-linked myotubular myopathy has been put on hold by the US Food and Drug administration due to deaths caused by progressive liver dysfunction (High-dose AAV gene therapy deaths. Nat Biotechnol 38, 910 (2020). https://doi.org/10.1038/s41587-020-0642-9). The dose delivered to these patients in the phase 2 trial was the highest of any AAV-based gene therapy to date (3 × 10¹⁴ vg per kg). Similarly, two deaths have been reported following treatment with spinal muscular atrophy gene therapy Zolgensma due to acute liver failure (High-dose AAV gene therapy deaths. Nat Biotechnol 38, 910 (2020). https://doi.org/10.1038/s41587-020-0642-9).

The use of cis-acting regulatory elements has been proposed to provide muscle specificity and activity. Typically, this concerns cis-regulatory enhancer sequences, i.e. nucleic acid sequences that act in cis to increase the activity of a promoter. Various muscle specific promoters are known in the art, typically obtained from genes that are expressed predominantly in the muscle, for example, such as those encoding for desmin, skeletal actin, heart α-actin, muscle creatine kinase (CKM), myosin heavy and light chains, and troponin T/I. The C5-12 promoter represents a known synthetic muscle promoter.

There remains a need in the art for systems to regulate gene expression in a specific manner with minimal off-target effects. In particular, there is a need for muscle-specific regulatory sequences which have minimal off-target effects. Such systems have the potential to minimise off-target effects of gene therapy vectors. This is particularly important for gene therapy vectors which have natural tropism for tissues other than the target tissues, such as AAV vectors which have natural tropism for liver cells and tissue. Furthermore, regulatory sequences of short length are desirable to minimise the proportion of a gene therapy vector taken up by regulatory sequences. This is particularly important for gene therapy vectors with limited capacity (payload) such as AAV vectors.

### Summary of the Invention

In a first aspect of the present invention, there is provided a synthetic muscle-specific cis-regulatory module (CRM) comprising CRE0145 or a functional variant thereof, DES_MT_enhancer_48bp or a functional variant thereof and at least one de-targeting element.

In some embodiments, the at least one de-targeting element is a liver de-targeting element.

In some embodiments, the synthetic muscle-specific cis-regulatory module (CRM) comprises CRE0145 or a functional variant thereof, DES_MT_enhancer_48bp or a functional variant thereof and a liver de-targeting element selected from the group consisting of:
- Liver de-targeting sequence 1 (SEQ ID NO: 13) or a functional variant thereof;
- Liver de-targeting sequence 2 (SEQ ID NO: 14) or a functional variant thereof;
- Liver de-targeting sequence 3 (SEQ ID NO: 17) or a functional variant thereof;
- ZBTB20 binding site (SEQ ID NO: 15) or a functional variant thereof;
- Mir122 miRNA target sequence 1 (SEQ ID NO: 16) or a functional variant thereof; and
- Mir122 miRNA target sequence 2 (SEQ ID NO: 19) or a functional variant thereof.

In some embodiments, a regulatory element, or functional variant thereof, as referred to herein may be selected from: CRE0145, DES_MT_enhancer_48bp, Liver de-targeting sequence 1, Liver de-targeting sequence 2, Liver de-targeting sequence 3, ZBTB20 binding site, Mir122 miRNA target sequence 1 and Mir122 miRNA target sequence 2.

In some embodiments, the synthetic muscle-specific CRM comprises a combination of regulatory elements, or functional variants thereof, selected from the group consisting of:
- CRE0145, DES_MT enhancer 48bp and Liver de-targeting sequence 1;
- CRE0145, DES_MT_enhancer_48bp and Liver de-targeting sequence 2;
- CRE0145, DES_MT_enhancer_48bp and Liver de-targeting sequence 3;
- CRE0145, Liver de-targeting sequence 3, DES_MT enhancer 48bp and Liver de-targeting sequence 3;
- CRE0145, DES_MT_enhancer_48bp and ZBTB20 binding site; and
- CRE0145, ZBTB20 binding site, DES_MT_enhancer_48bp and ZBTB20 binding site.

In some embodiments, the regulatory elements are present in the CRM in the recited order and adjacent to each other.

In any of the combinations of regulatory elements, or functional variants thereof, disclosed herein, the recited regulatory elements may be present in any order. In some preferred embodiments, the regulatory elements are present in the recited order (i.e. in an upstream to downstream order, with reference to their position with respect to an operably linked promoter element or gene).

In any of the combinations of regulatory elements, or functional variants thereof, disclosed herein, some or all of the recited CREs may suitably be positioned adjacent to one other in the CRM (i.e. without any intervening regulatory elements). The regulatory elements may be contiguous or non-contiguous (i.e. they can be positioned immediately adjacent to one another or they can be separated by a spacer or other sequence). In some preferred embodiments, the regulatory elements, or the functional variants thereof, are provided in the recited order and are adjacent to one another. For example, the synthetic muscle-specific CRM may comprise CRE0145, immediately upstream of DES_MT enhancer 48bp, immediately upstream of Liver de-targeting sequence 1 and so forth. In some embodiments, it is preferred that some or all of the CREs are contiguous.

In some embodiments of the present invention, the synthetic muscle-specific CRM comprises a CRM selected from the group consisting of: CRM_SP0525 (SEQ ID NO: 20) CRM_SP0526 (SEQ ID NO: 21), CRM_ SP0527 (SEQ ID NO: 22) and CRM_ SP0528 (SEQ ID NO: 9), or a functional variant of any thereof. Suitably, the functional variant of any of said CRMs comprises a sequence that is at least 70% identical to the reference synthetic muscle-specific CRM, more preferably at least 80%, 90%, 95% or 99% identical to the reference synthetic muscle-specific CRM (SEQ ID NO: 20, 21, 22 and 9).

In a further aspect of the present invention, there is provided a synthetic muscle-specific promoter comprising the CRM according to the previous aspect.

In some preferred embodiments, the CRM is operably linked to a promoter element. In some preferred embodiments, the promoter element is SCP1 (SEQ ID NO: 12) or a functional variant thereof or CRE0053 (SEQ ID NO: 26) or a functional variant thereof.

In some embodiments, the synthetic muscle-specific promoter comprises a synthetic muscle-specific CRM comprising CRE0145 or a functional variant thereof, DES_MT enhancer 48bp or a functional variant thereof and at least one liver de-targeting element selected from the group consisting of:
- Liver de-targeting sequence 1 (SEQ ID NO: 13) or a functional variant thereof;
- Liver de-targeting sequence 2 (SEQ ID NO: 14) or a functional variant thereof;
- Liver de-targeting sequence 3 (SEQ ID NO: 17) or a functional variant thereof; and
- ZBTB20 binding site (SEQ ID NO: 15) or a functional variant thereof;
operably linked to a promoter element SCP1 (SEQ ID NO: 12) or a functional variant thereof or CRE0053 (SEQ ID NO: 26) or a functional variant thereof.

In some embodiments, the synthetic muscle-specific cis-regulatory module (CRM) comprising CRE0145 or a functional variant thereof, DES_MT_enhancer_48bp or a functional variant thereof and ZBTB20 binding site (SEQ ID NO: 15) or a functional variant thereof.

In some embodiments, there is provided a synthetic muscle-specific promoter comprising: a synthetic muscle-specific CRM comprising a combination of regulatory elements, or functional variants thereof, selected from the group consisting of:
- CRE0145, DES_MT_enhancer_48bp and Liver de-targeting sequence 1;
- CRE0145, DES_MT_enhancer_48bp and Liver de-targeting sequence 2;
- CRE0145, DES_MT enhancer 48bp and Liver de-targeting sequence 3;
- CRE0145, DES_MT enhancer 48bp and ZBTB20 binding site;
- CRE0145, Liver de-targeting sequence 3, DES_MT_enhancer_48bp and Liver de-targeting sequence 3; and
- CRE0145, ZBTB20 binding site, DES_MT_enhancer_48bp and ZBTB20 binding site;
operably linked to a promoter element SCP1 (SEQ ID NO: 12) or a functional variant thereof or CRE0053 (SEQ ID NO: 26) or a functional variant thereof.

In some embodiments, the regulatory elements are present in the CRM in the recited order and adjacent to each other.

In any of the combinations of regulatory elements, or functional variants thereof, disclosed herein, the recited regulatory elements may be present in any order. In some preferred embodiments, the regulatory elements are present in the recited order (i.e. in an upstream to downstream order, with reference to their position with respect to an operably linked promoter element or gene).

In some embodiments, there is provided a synthetic muscle-specific promoter comprising or consisting of a cis-regulatory module (CRM) consisting or comprising CRM_SP0525 (SEQ ID NO: 20), CRM_SP0526 (SEQ ID NO: 21), CRM_ SP0527 (SEQ ID NO: 22) or CRM_ SP0528 (SEQ ID NO: 9) or a functional variant thereof. In some embodiments, the synthetic muscle-specific promoter comprises CRM consisting or comprising CRM_SP0525 (SEQ ID NO: 20), CRM_SP0526 (SEQ ID NO: 21), CRM_ SP0527 (SEQ ID NO: 22) or CRM_ SP0528 (SEQ ID NO: 9) or a functional variant thereof operably linked to a promoter element. The promoter element may be a minimal or proximal promoter. The proximal promoter is preferably a muscle-specific proximal promoter. The promoter element may be a promoter element which is broadly active across different tissues and cell types. In some embodiments, the promoter element is SCP1 (SEQ ID NO: 12). The promoter element may be a minimal promoter. In some embodiments, the promoter element is CRE0053 (SEQ ID NO: 26).

In some embodiments, there is provided a synthetic muscle-specific promoter comprising or consisting of a sequence according to any one of SEQ ID NOs: 1-4 or a functional variant thereof. In some embodiments, the synthetic muscle-specific promoter comprises a promoter selected from the group consisting of: SP0525 (SEQ ID NO: 1), SP0526 (SEQ ID NO: 2), SP0527 (SEQ ID NO: 3) and SP0528 (SEQ ID NO: 4), or a functional variant of any thereof. Suitably the functional variant of any said promoter comprises a sequence that is at least 70% identical to the reference synthetic muscle-specific promoter, more preferably at least 80%, 90%, 95% or 99% identical to the reference synthetic muscle-specific promoter (SEQ ID NO: 1-4).

In a further aspect, there is provided a synthetic muscle-specific promoter comprising or consisting of:
- CRE0145 or a functional variant thereof,
- DES_MT enhancer 48bp or a functional variant thereof,
- SCP1 or a functional variant thereof or CRE0053 or a functional variant thereof; and
- a de-targeting element, optionally a liver de-targeting element.

In some embodiments, there is provided a synthetic muscle-specific promoter comprising: CRE0145 or a functional variant thereof, DES_MT_enhancer_48bp or a functional variant thereof and at least a liver de-targeting element selected from the group consisting of:
- Liver de-targeting sequence 1 (SEQ ID NO: 13) or a functional variant thereof;
- Liver de-targeting sequence 2 (SEQ ID NO: 14) or a functional variant thereof;
- Liver de-targeting sequence 3 (SEQ ID NO: 17) or a functional variant thereof;
- ZBTB20 binding site (SEQ ID NO: 15) or a functional variant thereof;
- Mir122 miRNA target sequence 1 (SEQ ID NO: 16) or a functional variant thereof; and
- Mir122 miRNA target sequence 2 (SEQ ID NO: 19) or a functional variant thereof,
optionally operably linked to a promoter element SCP1 (SEQ ID NO: 12) or a functional variant thereof or promoter element CRE0053 (SEQ ID NO: 26) or a functional variant thereof.

In some embodiments, the synthetic muscle-specific promoter comprises SP0525 (SEQ ID NO: 1), SP0526 (SEQ ID NO: 2), SP0527 (SEQ ID NO: 3) or SP0528 (SEQ ID NO: 4) or a functional variant of any thereof. In some embodiments, the synthetic muscle-specific promoter comprises a sequence which is at least 70%, 75%, 80%, 85%, 90%, 95%, 96%, 97%, 98% or 99% identical to any one of SEQ ID NO 1-4.

In some embodiments, the muscle-specific promoters as set out above are operably linked to one or more additional regulatory elements. An additional regulatory element can, for example, enhance expression compared to a muscle-specific promoter which is not operably linked to the additional regulatory element. Generally, it is preferred that the additional regulatory element does not substantively reduce the specificity of a muscle-specific promoter.

For example, a synthetic muscle-specific promoter according to the present invention can be operably linked to a sequence encoding a UTR (e.g. a 5' and/or 3' UTR), and/or an intron, or suchlike. In a further aspect of the present invention, there is provided synthetic muscle-specific promoter SP0524 (SEQ ID NO: 7) operably linked to an intron.

In a further aspect of the present invention, there is provided a synthetic muscle-specific promoter comprising a liver de-targeting element. In some embodiments, there is provided a synthetic muscle-specific promoter comprising the minimal ZBTB20-binding site, suitably comprising SEQ ID NO: 15 or a functional variant thereof. In some embodiments, the synthetic muscle-specific promoter comprises sequence which is at least 70%, 75%, 80%, 85%, 90%, 95%, 96%, 97%, 98% or 99% identical to SEQ ID NO: 15. In some embodiments, the synthetic muscle-specific promoter comprises SEQ ID NO: 13 or a functional variant thereof. In some embodiments, the synthetic muscle-specific promoter comprises a sequence which is at least 70%, 75%, 80%, 85%, 90%, 95%, 96%, 97%, 98% or 99% identical to SEQ ID NO: 13. In some embodiments, the synthetic muscle-specific promoter comprises SEQ ID NO: 14 or a functional variant thereof. In some embodiments, the synthetic muscle-specific promoter comprises a sequence which is at least 70%, 75%, 80%, 85%, 90%, 95%, 96%, 97%, 98% or 99% identical to SEQ ID NO: 14. In some embodiments, the synthetic muscle-specific promoter comprises SEQ ID NO: 17 or a functional variant thereof. In some embodiments, the synthetic muscle-specific promoter comprises a sequence which is at least 70%, 75%, 80%, 85%, 90%, 95%, 96%, 97%, 98% or 99% identical to SEQ ID NO: 17. In some embodiments, the synthetic muscle-specific promoter comprising a liver de-targeting element has expression in the liver which is reduced by at least 5%, 10%, 15%, 20%, 25%, 30%, 35%, 40%, 45%, 50%, 55%, 60%, 65%, 70%, 75%, 80%, 85%, 90%, 95%, 99% compared to the equivalent synthetic muscle-specific promoter which does not comprise the liver de-targeting element. In some embodiments, the addition of the liver de-targeting element to the synthetic muscle-specific promoter does not impact its muscle-specific expression.

In a further aspect of the present invention, there is provided a synthetic CNS-specific promoter comprising a liver de-targeting element. In some embodiments, there is provided a synthetic CNS-specific promoter comprising the minimal ZBTB20-binding site (SEQ ID NO: 15) or a functional variant thereof. In some embodiments, the synthetic CNS-specific promoter comprises sequence which is at least 70%, 75%, 80%, 85%, 90%, 95%, 96%, 97%, 98% or 99% identical to SEQ ID NO: 15. In some embodiments, the synthetic CNS-specific promoter comprises SEQ ID NO: 13 or a functional variant thereof. In some embodiments, the synthetic CNS-specific promoter comprises a sequence which is at least 70%, 75%, 80%, 85%, 90%, 95%, 96%, 97%, 98% or 99% identical to SEQ ID NO: 13. In some embodiments, the synthetic CNS-specific promoter comprises SEQ ID NO: 14 or a functional variant thereof. In some embodiments, the synthetic CNS-specific promoter comprises a sequence which is at least 70%, 75%, 80%, 85%, 90%, 95%, 96%, 97%, 98% or 99% identical to SEQ ID NO: 14. In some embodiments, the synthetic CNS-specific promoter comprises SEQ ID NO: 17 or a functional variant thereof. In some embodiments, the synthetic CNS-specific promoter comprises a sequence which is at least 70%, 75%, 80%, 85%, 90%, 95%, 96%, 97%, 98% or 99% identical to SEQ ID NO: 17. In some embodiments, the synthetic CNS-specific promoter comprising a liver de-targeting element has expression in the liver which is reduced by at least 5%, 10%, 15%, 20%, 25%, 30%, 35%, 40%, 45%, 50%, 55%, 60%, 65%, 70%, 75%, 80%, 85%, 90%, 95%, 99% compared to the equivalent synthetic CNS-specific promoter which does not comprise the liver de-targeting element. In some embodiments, the addition of the liver de-targeting element to the synthetic CNS-specific promoter does not impact its CNS-specific expression.

In a further aspect of the present invention, there is provided a synthetic kidney-specific promoter comprising a liver de-targeting element. In some embodiments, there is provided a synthetic kidney-specific promoter comprising the minimal ZBTB20-binding site (SEQ ID NO: 15) or a functional variant thereof. In some embodiments, the synthetic kidney-specific promoter comprises sequence which is at least 70%, 75%, 80%, 85%, 90%, 95%, 96%, 97%, 98% or 99% identical to SEQ ID NO: 15. In some embodiments, the synthetic kidney-specific promoter comprises SEQ ID NO: 13 or a functional variant thereof. In some embodiments, the synthetic kidney-specific promoter comprises a sequence which is at least 70%, 75%, 80%, 85%, 90%, 95%, 96%, 97%, 98% or 99% identical to SEQ ID NO: 13. In some embodiments, the synthetic kidney-specific promoter comprises SEQ ID NO: 14 or a functional variant thereof. In some embodiments, the synthetic kidney-specific promoter comprises a sequence which is at least 70%, 75%, 80%, 85%, 90%, 95%, 96%, 97%, 98% or 99% identical to SEQ ID NO: 14. In some embodiments, the synthetic kidney-specific promoter comprises SEQ ID NO: 17 or a functional variant thereof. In some embodiments, the synthetic kidney-specific promoter comprises a sequence which is at least 70%, 75%, 80%, 85%, 90%, 95%, 96%, 97%, 98% or 99% identical to SEQ ID NO: 17. In some embodiments, the synthetic kidney-specific promoter comprising a liver de-targeting element has expression in the liver which is reduced by at least 5%, 10%, 15%, 20%, 25%, 30%, 35%, 40%, 45%, 50%, 55%, 60%, 65%, 70%, 75%, 80%, 85%, 90%, 95%, 99% compared to the equivalent synthetic kidney-specific promoter which does not comprise the liver de-targeting element. In some embodiments, the addition of the liver de-targeting element to the synthetic kidney-specific promoter does not impact its kidney-specific expression.

In a further aspect of the present invention, there is provided a synthetic lung-specific promoter comprising a liver de-targeting element. In some embodiments, there is provided a synthetic lung-specific promoter comprising the minimal ZBTB20-binding site (SEQ ID NO: 15) or a functional variant thereof. In some embodiments, the synthetic lung-specific promoter comprises sequence which is at least 70%, 75%, 80%, 85%, 90%, 95%, 96%, 97%, 98% or 99% identical to SEQ ID NO: 15. In some embodiments, the synthetic lung-specific promoter comprises SEQ ID NO: 13 or a functional variant thereof. In some embodiments, the synthetic lung-specific promoter comprises a sequence which is at least 70%, 75%, 80%, 85%, 90%, 95%, 96%, 97%, 98% or 99% identical to SEQ ID NO: 13. In some embodiments, the synthetic lung-specific promoter comprises SEQ ID NO: 14 or a functional variant thereof. In some embodiments, the synthetic lung-specific promoter comprises a sequence which is at least 70%, 75%, 80%, 85%, 90%, 95%, 96%, 97%, 98% or 99% identical to SEQ ID NO: 14. In some embodiments, the synthetic lung-specific promoter comprises SEQ ID NO: 17 or a functional variant thereof. In some embodiments, the synthetic lung-specific promoter comprises a sequence which is at least 70%, 75%, 80%, 85%, 90%, 95%, 96%, 97%, 98% or 99% identical to SEQ ID NO: 17. In some embodiments, the synthetic lung-specific promoter comprising a liver de-targeting element has expression in the liver which is reduced by at least 5%, 10%, 15%, 20%, 25%, 30%, 35%, 40%, 45%, 50%, 55%, 60%, 65%, 70%, 75%, 80%, 85%, 90%, 95%, 99% compared to the equivalent synthetic lung-specific promoter which does not comprise the liver de-targeting element. In some embodiments, the addition of the liver de-targeting element to the synthetic lung-specific promoter does not impact its lung-specific expression.

In a further aspect of the present invention, there is provided an expression cassette comprising a synthetic muscle-specific promoter according to any aspect of the present invention operably linked to a sequence encoding an expression product, suitably a gene, e.g. a transgene.

In some embodiments the expression product is a therapeutic expression product. In some preferred embodiments, the therapeutic expression product is suitable for use in treating a disease or condition associated with aberrant gene expression, optionally in the muscle (i.e. muscular disease), optionally in cardiac muscle and/or in skeletal muscle. In some preferred embodiments, therapeutic expression products include those useful in the treatment of muscle diseases.

The therapeutic expression product may be a therapeutic expression product useful in the treatment of any condition where expression in muscle may be of use, e.g. for treatment of a muscle condition or the treatment of a condition where secretion of the therapeutic expression product from the muscle may be desirable.

In a further aspect, there is provided an expression cassette comprising a synthetic muscle-specific promoter comprising CRE0145 or a functional variant thereof and DES_MT_enhancer_48bp or functional variant thereof, wherein the synthetic promoter is operably linked to a sequence encoding an expression product and a target sequence for miR122.

In some embodiments, the synthetic muscle-specific promoter further comprises SCP1 (SEQ ID NO: 12) or a functional variant thereof or CRE0053 (SEQ ID NO: 26) or a functional variant thereof.

In some preferred embodiments, the expression cassette comprises SP0524 (SEQ ID NO: 7) operably linked to a sequence encoding an expression product and a target sequence for miR122.

In some preferred embodiments, the target sequence for miR122 comprises or consists of SEQ ID NO: 16 or SEQ ID NO: 19 or a functional variant thereof. In some preferred embodiments, the target sequence for miR122 comprises or consists of a sequence which is at least 70%, 75%, 80%, 85%, 90%, 95%, 96%, 97%, 98% or 99% identical to SEQ ID NO: 16 or SEQ ID NO: 19.

In a further aspect, there is provided a vector comprising a synthetic muscle-specific promoter or an expression cassette according to the present invention. In some embodiments, the vector is a gene therapy vector. In some embodiments the vector is an expression vector. In some embodiments the vector is a viral vector. In some embodiments the vector is a gene therapy vector, suitably an AAV vector, an adenoviral vector, a retroviral vector or a lentiviral vector. AAV vectors are of particular interest. AAV vectors may be selected from the group consisting of AAV2, AAV6, AAV8, AAV9, BNP116, rh10, AAV2.5, AAV2i8, AAVDJ8 and AAV2G9, or derivatives thereof. AAV serotype 9 (AAV9) has been noted to achieve efficient transduction in cardiac and skeletal muscle, and thus AAV9 and derivatives thereof represent one non-limiting example of a suitable AAV vector. In some embodiments, the rAAV vector is a AAV3b serotype, including, but not limited to, an AAV3b265D virion, an AAV3b265D549A virion, an AAV3b549A virion, an AAV3bQ263Y virion, or an AAV3bSASTG virion (i.e., a virion comprising a AAV3b capsid comprising Q263A/T265 mutations). In some embodiments, the virion can be rational haploid, or a chimeric or any mutant, such as capsids can be tailored for increased uptake at a desired location, e.g., the heart. Other capsids can include capsids from any of the known AAV serotypes, including AAV1, AAV3, AAV4, AAV5, AAV7, AAV10, etc. In some preferred embodiments, the AAV vector is AAV2i8.

The vector according to the present invention may be an AAV vector comprising a nucleic acid encoding a therapeutic expression product for treatment of heart failure, wherein the nucleic acid is operatively linked to a muscle-specific promoter.

In a further aspect of the present invention, there is provided a gene therapy vector comprising a synthetic promoter comprising SEQ ID NO: 15 or a functional variant thereof. In some embodiments, the gene therapy vector comprises a synthetic promoter comprising a sequence which is at least 70%, 75%, 80%, 85%, 90%, 95%, 96%, 97%, 98% or 99% identical to SEQ ID NO: 15. In some embodiments, the gene therapy vector comprises a synthetic promoter comprising SEQ ID NO: 13 or a functional variant thereof. In some embodiments, the gene therapy vector comprises a synthetic promoter comprising a sequence which is at least 70%, 75%, 80%, 85%, 90%, 95%, 96%, 97%, 98% or 99% identical to SEQ ID NO: 13. In some embodiments, the gene therapy vector comprises a synthetic promoter comprising SEQ ID NO: 14 or a functional variant thereof. In some embodiments, the gene therapy vector comprises a synthetic promoter comprising a sequence which is at least 70%, 75%, 80%, 85%, 90%, 95%, 96%, 97%, 98% or 99% identical to SEQ ID NO: 14. In some embodiments, the gene therapy vector comprises a synthetic promoter comprising SEQ ID NO: 17 or a functional variant thereof. In some embodiments, the gene therapy vector comprises a synthetic promoter comprising a sequence which is at least 70%, 75%, 80%, 85%, 90%, 95%, 96%, 97%, 98% or 99% identical to SEQ ID NO: 17.

In some preferred embodiments, the gene therapy vector is a viral vector, such as a retroviral, lentiviral, adenoviral, or adeno-associated viral (AAV) vector. In some preferred embodiments the vector is an AAV vector. Suitable AAV vectors are described above.

In a further aspect, there is provided a gene therapy AAV vector comprising an expression cassette, the expression cassette comprising a synthetic promoter operably linked to a sequence encoding an expression product and a target sequence for miR122.

In some embodiments, the gene therapy AAV vector is AAV2. AAV2 has been noted to achieve efficient transduction in liver and muscle. In some embodiments, the gene therapy AAV vector is AAV9. AAV9 has been noted to achieve efficient transduction in muscle and CNS and is currently used in a clinical context. In some embodiments, the gene therapy AAV vector is AAV5. AAV5 has been noted to achieve efficient transduction in lung. In some embodiments, the gene therapy AAV vector is AAV8. AAV8 has been noted to achieve efficient transduction in muscle and is currently used in a clinical context. A target sequence for miR122 is expected to reduce liver expression of the expression in the gene therapy AAV vector.

In some embodiments, the target sequence for miR122 comprises or consists of Mir122 miRNA target sequence 1. In some embodiments, the target sequence for miR122 comprises or consists of SEQ ID NO: 16 or a functional variant thereof. In some embodiments, the target sequence for miR122 comprises or consists of a sequence which is at least 70%, 75%, 80%, 85%, 90%, 95%, 96%, 97%, 98% or 99% identical to SEQ ID NO: 16. In some embodiments, the target sequence for miR122 comprises or consists of Mir122 miRNA target sequence 2. In some embodiments, the target sequence for miR122 comprises or consists of SEQ ID NO: 19. In some embodiments, the target sequence for miR122 comprises or consists of a sequence which is at least 70%, 75%, 80%, 85%, 90%, 95%, 96%, 97%, 98% or 99% identical to SEQ ID NO: 19.

In some embodiments, the gene therapy AAV vector comprises a muscle-specific promoter operably linked to a sequence encoding an expression product (e.g. a gene of interest) and mir122 miRNA target sequence 1 (SEQ ID NO: 16). In some embodiments, the gene therapy AAV vector comprises a muscle-specific promoter operably linked to a sequence encoding an expression product (e.g. a gene of interest) and Mir122 miRNA target sequence 2 (SEQ ID NO: 19). In some preferred embodiments, the elements are present in the recited order and adjacent to each other.

In some embodiments, the gene therapy AAV vector comprises a muscle-specific promoter operably linked to a sequence encoding an expression product (e.g. a gene of interest), T2A peptide, a further expression product (e.g. a second gene of interest) and mir122 miRNA target sequence 1 (SEQ ID NO: 16). In some embodiments, the gene therapy AAV vector comprises a muscle-specific promoter operably linked to a sequence encoding an expression product (e.g. a gene of interest), T2A peptide, a further expression product (e.g. a second gene of interest) and Mir122 miRNA target sequence 2 (SEQ ID NO: 19). In some preferred embodiments, the elements are present in the recited order and adjacent to each other.

In some embodiments, the gene therapy AAV vector comprises SP0524 (SEQ ID NO: 7) operably linked to a sequence encoding an expression product (e.g. a gene of interest) and mir122 miRNA target sequence 1 (SEQ ID NO: 16). In some embodiments, the gene therapy AAV vector comprises a SP0524 (SEQ ID NO: 7) operably linked to a sequence encoding an expression product (e.g. a gene of interest) and Mir122 miRNA target sequence 2 (SEQ ID NO: 19). In some preferred embodiments, the elements are present in the recited order and adjacent to each other.

In some embodiments, the gene therapy AAV vector comprises SP0524 (SEQ ID NO: 7) operably linked to a sequence encoding an expression product (e.g. a gene of interest), T2A peptide, a further expression product (e.g. a second gene of interest) and mir122 miRNA target sequence 1 (SEQ ID NO: 16). In some embodiments, the gene therapy AAV vector comprises SP0524 (SEQ ID NO: 7) operably linked to a sequence encoding an expression product (e.g. a gene of interest), T2A peptide, a further expression product (e.g. a second gene of interest) and Mir122 miRNA target sequence 2 (SEQ ID NO: 19). In some preferred embodiments, the elements are present in the recited order and adjacent to each other.

In a further aspect, there is provided a virion (viral particle) comprising a vector, suitably a viral vector, according to the present invention. In some embodiments the virion is an AAV virion. Suitable virions are described above.

In a further aspect, there is provided a pharmaceutical composition comprising a synthetic muscle-specific promoter, expression cassette, vector or virion according to the present invention.

In a further aspect, there is provided a synthetic muscle-specific promoter, expression cassette, vector, virion or pharmaceutical composition according to the present invention for use in therapy, i.e. the prevention or treatment of a medical condition or disease. In some embodiments, the synthetic muscle-specific promoter, expression cassette, vector, virion or pharmaceutical composition according to the present invention are for use in therapy of subject in need thereof. Suitably the condition or disease is associated with aberrant gene expression, optionally aberrant gene expression in muscle cells (myocytes) or tissue. Suitably the condition or disease is associated with aberrant gene expression, in cardiac muscle cells or heart tissue. Suitably the condition or disease is associated with aberrant gene expression in skeletal muscle or tissue. Suitably, there is provided a synthetic muscle-specific promoter, expression cassette, vector, virion or pharmaceutical composition according to the present invention for use in expression of a therapeutic expression product in the skeletal and/or cardiac muscle.

In one embodiment, the disease may be cardiovascular condition or heart disease and disorders. In one embodiment, the disease may be heart failure such as congestive heart failure. In one embodiment, the disease may be selected from ischemia, arrhythmia, myocardial infarction (MI), abnormal heart contractility, non-ischemic cardiomyopathy, peripheral arterial occlusive disease, and abnormal Ca²⁺ metabolism, and combinations thereof. In some embodiments, the disease may be selected from the group of: congestive heart failure, cardiomyopathy, myocardial infarction, tissue ischemia, cardiac ischemia, vascular disease, acquired heart disease, congenital heart disease, atherosclerosis, dysfunctional conduction systems, dysfunctional coronary arteries, pulmonary heart hypertension. In some embodiments, the disease may be selected from congestive heart failure, coronary artery disease, myocardial infarction, myocardial ischemia, atherosclerosis, cardiomyopathy, idiopathic cardiomyopathy, cardiac arrhythmias, muscular dystrophy, muscle mass abnormality, muscle degeneration, infective myocarditis, drug- or toxin-induced muscle abnormalities, hypersensitivity myocarditis, an autoimmune endocarditis and congenital heart disease.

Suitably the use is for gene therapy, preferably for use in treatment of a disease involving aberrant gene expression. Suitably the gene therapy involves expression of a therapeutic expression product in muscle cells or tissue, suitably in cardiac muscle cells or heart tissue, or suitably in skeletal muscle cells or tissue.

Suitably, the subject in need of therapy will display symptoms characteristic of a cardiovascular condition, e.g., heart disease or heart failure as discussed above. The medical use typically comprises ameliorating the symptoms displayed by the subject in need thereof, by expressing the therapeutic amount of the therapeutic product. In some embodiments, the expression cassette comprises a gene encoding an inhibitor of the protein phosphate 1 (PP1), operably linked to a cardiac muscle-specific promoter. The therapy suitably comprises expressing a therapeutic amount of the inhibitor of PP1 in the heart tissue of said subject. Suitably, expressing a therapeutic amount of the inhibitor of PP1 in the heart tissue reduces the symptoms of heart failure or a heart disorder of a subject. Suitably, expressing a therapeutic amount of the inhibitor of PP1 in the heart tissue may attenuate cardiac remodelling, improve exercise capacity, or improve cardiac contractility. Suitably, expressing a therapeutic amount of the inhibitor of PP1 in the heart tissue may result in myocyte shortening, lowering of the time constant for relaxation, and accelerating calcium signal decay, improving the end-systolic pressure dimension relationship and combinations thereof.

In a further aspect, there is provided a cell comprising a synthetic muscle-specific promoter, expression cassette, vector, or virion of the present invention. In some embodiments the cell is a eukaryotic cell, optionally a mammalian cell, optionally a human cell. Suitably the cell can be a muscle cell, optionally wherein the cell is a human muscle cell. Suitably, the cell may be a human skeletal muscle cell or human cardiac muscle cell. The synthetic muscle-specific promoter, expression cassette can be episomal or can be in the genome of the cell.

In a further aspect, there is provided a synthetic muscle-specific CRM, synthetic muscle-specific promoter, expression cassette, vector, virion or pharmaceutical composition as described herein for use in the manufacture of a pharmaceutical composition for the treatment of a medical condition or disease.

In a further aspect, there is provided a method for producing an expression product, the method comprising providing a synthetic muscle-specific expression cassette of the present invention in a muscle cell and expressing the expression product, suitably a gene, present in the synthetic muscle-specific expression cassette. The method can be *in vitro* or ex *vivo,* or it can be *in vivo.* In some embodiments the method is a bioprocessing method. In one embodiment, the muscle cell is a cardiac muscle cell. In one embodiment, the muscle cell is a skeletal muscle cell.

In a further aspect, there is provided a method of expressing an expression product in a muscle cell, the method comprising introducing into the muscle cell a synthetic muscle-specific expression cassette, vector or virion as described herein. In one embodiment, the muscle cell is a cardiac muscle cell. In one embodiment, the muscle cell is a skeletal muscle cell.

In a further aspect, there is provided a method of therapy of a subject, preferably a human, in need thereof, the method comprising:
- administering to the subject an expression cassette, vector, virion or pharmaceutical composition as described herein, which comprises a sequence encoding a therapeutic product operably linked to a synthetic muscle-specific promoter according to the present invention; and
- expressing a therapeutic amount of the therapeutic product in the muscle of said subject.

In one embodiment, the muscle cell is a cardiac muscle cell. In one embodiment, the muscle cell is a skeletal muscle cell. Suitably, the method of therapy of a subject comprises expression of the therapeutic amount of the therapeutic product in the cardiac and/or skeletal muscle.

In some embodiments the method comprises:
- introducing into the muscle of the subject an expression cassette, vector, virion or pharmaceutical composition as described herein, which comprises an expression product, suitably a gene encoding a therapeutic product; and
- expressing a therapeutic amount of the therapeutic product in the muscle of said subject.

In one embodiment, the muscle cell is a cardiac muscle cell. In one embodiment, the muscle cell is a skeletal muscle cell. Suitably, the method comprises expression of the therapeutic amount of the therapeutic product in the cardiac and/or skeletal muscle of said subject.

Suitably the method comprises administering a vector, virion or pharmaceutical composition as described herein to the subject. In some preferred embodiments the vector is a viral gene therapy vector, preferably an AAV vector.

In one embodiment, any of the aspects herein relating to a muscle-specific promoter may equally relate to a CNS, kidney or lung specific promoter as defined hereinabove.

Further features and embodiments of the present invention will now be described under the following sections. Any feature or embodiment in any section may be combined with any other feature or embodiment, or with any aspect of the invention, in any workable combination.

### Detailed Description of Embodiments of the Invention and Examples

### Muscle-specific and muscle-specificity

The CREs, CRMs, promoter elements and synthetic promoters of the present invention can be active in various muscle tissues, particularly but not exclusively in skeletal muscle and/or cardiac muscle. The CREs, CRMs, promoter elements and synthetic promoters of the present invention which are active in at least one muscle tissue type or at least one muscle cell type may be referred to as 'muscle-specific'. For ease, CREs, CRMs, promoter elements and synthetic promoters of the present invention can be further subdivided in subtypes depending on whether they are predominantly active in skeletal or cardiac muscle.

In some embodiments the CREs, CRMs, promoter elements and synthetic promoters of the present invention are active predominantly in skeletal muscle and less active or not active in cardiac muscle. These CREs, CRMs, promoter elements and synthetic promoters of the present invention are called 'skeletal muscle-specific'. In some embodiments the CREs, CRMs, promoter elements and synthetic promoters of the present invention are skeletal muscle-specific.

In some embodiments the CREs, CRMs, promoter elements and synthetic promoters of the present invention are active predominantly in cardiac muscle and less active or not active in skeletal muscle. These CREs, CRMs, promoter elements and synthetic promoters of the present invention are called 'cardiac muscle-specific'. In some embodiments the CREs, CRMs, promoter elements and synthetic promoters of the present invention are cardiac muscle-specific.

In some embodiments, muscle-specific CREs, CRMs, promoter elements and synthetic promoters which are active in both skeletal muscle and cardiac muscle are preferred. These CREs, CRMs, promoter elements and synthetic promoters may be preferred when promoter activity is required in both the skeletal muscle and the heart (in the cardiac muscles). Examples of muscle-specific promoters active in both skeletal and cardiac muscle include SP0524 (SEQ ID NO: 7).

In some embodiments, skeletal muscle-specific CREs, CRMs, promoter elements and synthetic promoters are preferred. These CREs, CRMs, promoter elements and synthetic promoters may be preferred when promoter activity is required in the skeletal muscle with little or no activity in the heart (in the cardiac muscles).

The skeletal muscle-specific promoters may be active in fast-twitch muscles and/or slow-twitch muscles. In some embodiments, skeletal muscle-specific CREs, CRMs, promoter elements and synthetic promoters active in fast-twitch muscles may be preferred. In some embodiments, skeletal muscle-specific CREs, CRMs, promoter elements and synthetic promoters active in slow-twitch muscles may be preferred. In some embodiments, skeletal muscle-specific CREs, CRMs, promoter elements and synthetic promoters active both in slow-twitch and fast-twitch muscles may be preferred.

In some embodiments, cardiac muscle-specific CREs, CRMs, promoter elements and synthetic promoters are preferred. These CREs, CRMs, promoter elements and synthetic promoters may be preferred when promoter activity is required in the heart (in the cardiac muscles) with little or no activity in the skeletal muscles.

The cardiac muscle-specific CREs, CRMs, promoter elements and synthetic promoters of the present invention can be active in various cells of the heart. The predominant cell types in the heart are ventricular cardiomyocytes, atrial cardiomyocytes, cardiac fibroblasts, or endothelial cells (EC) in the heart, as well as peri-vascular cells and pacemaker cells. Additionally, the cardiac muscle-specific CREs, CRMs, promoter elements and synthetic promoters of the present invention can be active in various regions of the heart, such as, for example, activity in any or all of the following heart regions: aortic arch arteries (AA); aorta; cardiomyocytes (CM); endothelial or endocardial cells (ECs); inferior caval vein (ICV); interventricular septum (IVS); left atrium (LA); left superior caval vein (LSCV); left ventricle (LV); outflow tract (OT); pulmonary arteries (PO); proepicardial organ (PEO); pulmonary vein (PV); right atrium (RA); right superior caval vein (RSCV); right ventricle (RV); superior caval vein (SCV); cardiac smooth muscle cells (SMs).

### Cis-requlatorv elements and functional variants thereof

Disclosed herein are various CREs that can be used in the construction of muscle-specific promoters. These CREs are generally derived from genomic promoter and enhancer sequences, but they are used herein in contexts quite different from their native genomic environment. Generally, the CREs constitute small parts of much larger genomic regulatory domains, which control expression of the genes with which they are normally associated. It has been surprisingly found that these CREs, many of which are very small, can be isolated from their normal environment and retain muscle-specific regulatory activity when used to construct various synthetic promoters. This is surprising because the removal of a regulatory sequence from the complex and "three dimensional" natural context in the genome often results in a significant loss of activity, so there is no reason to expect a given CRE to retain the levels of activity observed once removed from its natural environment. Combinations of these CREs have been tested and found to be highly effective at enhancing muscle-specific promoter activity when combined with minimal and proximal promoters. It should be noted that the sequences of the CREs of the present invention can be altered without causing a substantial loss of activity. Functional variants of the CREs can be prepared by modifying the sequence of the CREs, provided that modifications which are significantly detrimental to activity of the CRE are avoided. In view of the information provided in the present disclosure, modification of CREs to provide functional variants is straightforward. Moreover, the present disclosure provides methodologies for simply assessing the functionality of any given CRE variant. Functional variants for each CRE are discussed below.

The relatively small size of certain CREs according to the present invention is advantageous because it allows for the CREs, more specifically promoters containing them, to be provided in vectors while taking up the minimal amount of the payload of the vector. This is particularly important when a CRE is used in a vector with limited capacity, such as an AAV-based vector.

CREs disclosed herein comprise certain muscle-specific transcription factor binding sites (TFBS). It is generally desired that in functional variants of the CREs these muscle-specific TFBS remain functional. The skilled person is well aware that TFBS sequences can vary yet retain functionality. In view of this, the sequence for a TFBS is typically illustrated by a consensus sequence from which some degree of variation is typically present. Further information about the variation that occurs in a TFBS can be illustrated using a positional weight matrix (PWM), which represents the frequency with which a given nucleotide is typically found at a given location in the consensus sequence. Details of TF consensus sequences and associated positional weight matrices can be found in, for example, the Jaspar or Transfac databases http://jaspar.genereg.net/ and http://gene-regulation.com/pub/databases.html). This information allows the skilled person to modify the sequence in any given TFBS of a CRE in a manner which retains, and in some cases even increases, CRE functionality. In view of this the skilled person has ample guidance on how the TFBS for any given transcription factor (TF) can be modified, while maintaining ability to bind the desired TF; the Jaspar system will, for example, score a putative TFBS based on its similarity to a given PWM. Furthermore, CREs can be scanned against all PWM from JASPAR database to identify/analyse all TFBS. The skilled person can of course find additional guidance in the literature, and, moreover, routine experimentation can be used to confirm TF binding to a putative TFBS in any variant CRE. It will be apparent that significant sequence modification in a CRE, even within TFBS in a CRE, can be made while retaining function.

A functional variant of a CRE can comprise substitutions, deletions and/or insertions compared to a reference CRE, provided they do not render the CRE substantially non-functional.

CRE0145 (SEQ ID NO: 10) and DES_MT enhancer 48bp (SEQ ID NO: 11) are muscle-specific cis-regulatory elements (CREs). These CREs have been found to provide significant muscle-specific enhancer activity when combined with a suitable promoter element and/or when added to a suitable synthetic promoter.

### Promoter elements and functional variants thereof

The CRMs of the present invention can be used in combination with a wide range of suitable minimal promoters or muscle-specific proximal promoters. The minimal promoters and proximal promoters are collectively called promoter elements.

Functional variants of promoter elements include sequences which vary from the reference promoter element, but which substantially retain activity as muscle-specific promoter element. It will be appreciated by the skilled person that it is possible to vary the sequence of a promoter element while retaining its ability to promote expression. A functional variant of a promoter element can comprise substitutions, deletions and/or insertions compared to a reference promoter element, provided they do not render the promoter element substantially non-functional.

A functional variant of a promoter element can be viewed as a promoter element which, when substituted in place of a reference promoter element in a synthetic promoter, substantially retains its activity. For example, a muscle-specific synthetic promoter which comprises a functional variant of a given promoter element preferably retains at least 80% of its activity, more preferably at least 90% of its activity, more preferably at least 95% of its activity, and yet more preferably 100% of its activity (compared to the reference promoter comprising the unmodified promoter element).

Suitably, functional variants of a promoter element retain a significant level of sequence identity to a reference promoter element. Suitably functional variants comprise a sequence that is at least 70% identical to the reference promoter element, more preferably at least 80%, 90%, 91%, 92%, 93%, 94%, 95%, 96%, 97%, 98% or 99% identical to the reference promoter element.

Retention of activity can be assessed by comparing expression of a suitable reporter under the control of the reference promoter with an otherwise identical promoter comprising the substituted promoter element under equivalent conditions. Suitable methods for assessing muscle-specific promoter activity are disclosed herein, e.g. in the examples.

SCP1 (SEQ ID NO: 12) is a promoter element. This promoter element has been found to provide significant muscle-specific activity when combined with cis-regulatory elements CRE0145 (SEQ ID NO: 10) and DES_MT_enhancer_48bp (SEQ ID NO: 11).

CRE0053 (SEQ ID NO: 26) is a promoter element, suitably a minimal promoter. This promoter element has been found to provide significant muscle-specific activity when combined with cis-regulatory elements CRE0145 (SEQ ID NO: 10) and DES_MT enhancer 48bp (SEQ ID NO: 11), suitably significant cardiac muscle-specific activity.

### De-targeting element and liver de-targeting element

A de-targeting element may be added to a synthetic promoter in order to reduce expression from the synthetic promoter in specific tissues or cells. In some embodiments, the de-targeting element is a binding site for a protein which is highly expressed in the specific tissue or cell. In some embodiments, the de-targeting element is a liver de-targeting element.

In one embodiment, the liver de-targeting element reduces expression of the synthetic promoter comprising the element in the liver by at least 10% as compared to an appropriate control. In one embodiment, the liver de-targeting element reduces expression of the synthetic promoter comprising the element in the liver by at least 5%, 10%, 15%, 20%, 25%, 30%, 35%, 40%, 45%, 50%, 55%, 60%, 65%, 70%, 75%, 80%, 85%, 90%, 95%, 99%, or more as compared to an appropriate control. As used herein, "appropriate control" refers to the expression level of the synthetic promoter not containing the de-targeting element in the liver in an otherwise identical sample. One skilled in the art can assess the expression level of a given synthetic promoter (with or without the de-targeting element) in the liver using standard techniques, for example, using PCR-based assays to measure mRNA levels in liver cells and/or tissue samples.

Alpha-fetoprotein (AFP) gene is activated in fetal liver but repressed after birth. ZBTB20 is a zinc finger protein which acts as a transcriptional repressor of AFP promoter mediated activity in liver (Xie Z, Zhang H, Tsai W, Zhang Y, Du Y, Zhong J, Szpirer C, Zhu M, Cao X, Barton MC, Grusby MJ, Zhang WJ. Zinc finger protein ZBTB20 is a key repressor of alpha-fetoprotein gene transcription in liver. Proc Natl Acad Sci U S A. 2008 Aug 5;105(31):10859-64. doi: 10.1073/pnas.0800647105. Epub 2008 Jul 31. PMID: 18669658; PMCID: PMC2504784). Different regions to which ZBTB20 binds have been identified in the literature (Zhang, H., Cao, D., Zhou, L. et al. ZBTB20 is a sequence-specific transcriptional repressor of alpha-fetoprotein gene. Sci Rep 5, 11979 (2015). https://doi.org/10.1038/srep11979). Notably, Zhang, et al., 2015 teach away from reducing the size of the -151/-53 region of AFP (which they identified as a region to which ZBTB20 binds) as this reduces promoter activity.

SEQ ID NO: 15 shows a minimal ZBTB20 binding site. Liver de-targeting sequence 1 (SEQ ID NO: 13), Liver de-targeting sequence 2 (SEQ ID NO: 14) and Liver de-targeting sequence 3 (SEQ ID NO: 17) comprise the minimal ZBTB20 binding site.

In some embodiments, the de-targeting element is a binding site for a miRNA which is highly expressed in the specific tissue or cell. In some embodiments, the de-targeting element is a liver de-targeting element.

miR122 is highly expressed in the liver and miR122 target sequence is effective at reducing liver expression (Qiao C, Yuan Z, Li J, He B, Zheng H, Mayer C, Li J, Xiao X. Liver-specific microRNA-122 target sequences incorporated in AAV vectors efficiently inhibits transgene expression in the liver. Gene Ther. 2011 Apr;18(4):403-10. doi: 10.1038/gt.2010.157. Epub 2010 Dec 9. PMID: 21150938; PMCID: PMC3686499).

SEQ ID NO: 19 shows a minimal Mir122 miRNA target sequence. Mir122 miRNA target sequence 1 (SEQ ID NO: 16) comprises the minimal Mir122 miRNA target sequence three times.

Functional variants of de-targeting element or liver de-targeting element include sequences which vary from the reference de-targeting element or liver de-targeting element, but which substantially retain activity as de-targeting element or liver de-targeting element. It will be appreciated by the skilled person that it is possible to vary the sequence of a de-targeting element while retaining its ability to reduce expression from the synthetic promoter in specific tissues or cells. It will be appreciated by the skilled person that it is possible to vary the sequence of a liver de-targeting element while retaining its ability to reduce expression from the synthetic promoter in liver tissues or cells. A functional variant of a de-targeting element or liver de-targeting element can comprise substitutions, deletions and/or insertions compared to a reference de-targeting element or liver de-targeting element, provided they do not render the de-targeting element or liver de-targeting element substantially non-functional.

A functional variant of a de-targeting element or liver de-targeting element can be viewed as de-targeting element or liver de-targeting element which, when substituted in place of a reference de-targeting element or liver de-targeting element in a synthetic promoter, substantially retains its activity. For example, a muscle-specific synthetic promoter which comprises a functional variant of a given de-targeting element or liver de-targeting element preferably retains at least 80% of its activity, more preferably at least 90% of its activity, more preferably at least 95% of its activity, and yet more preferably 100% of its activity (compared to the reference promoter comprising the unmodified de-targeting element or liver de-targeting element).

Suitably, functional variants of a de-targeting element or liver de-targeting element retain a significant level of sequence identity to a reference de-targeting element or liver de-targeting element. Suitably functional variants comprise a sequence that is at least 70% identical to the reference de-targeting element or liver de-targeting element, more preferably at least 80%, 90%, 91%, 92%, 93%, 94%, 95%, 96%, 97%, 98% or 99% identical to the reference de-targeting element or liver de-targeting element.

Retention of activity can be assessed by comparing expression of a suitable reporter under the control of the reference promoter with an otherwise identical promoter comprising the substituted de-targeting element or liver de-targeting element under equivalent conditions. Suitable methods for assessing muscle-specific promoter activity are disclosed herein, e.g. in the examples.

### Synthetic Muscle-Specific CRMs and Functional Variants Thereof

Various synthetic muscle-specific CRMs are disclosed herein that can be used in the constructions of synthetic muscle-specific promoters. CRMs of the present invention can be used in combination with a wide range of suitable minimal promoters or muscle-specific proximal promoters.

Functional variants of a CRM include sequences which vary from the reference CRM element, but which substantially retain activity as muscle-specific CRMs. It will be appreciated by the skilled person that it is possible to vary the sequence of a CRM while retaining its ability to recruit suitable muscle-specific transcription factors (TFs) and thereby enhance expression. A functional variant of a CRM can comprise substitutions, deletions and/or insertions compared to a reference CRM, provided they do not render the CRM substantially non-functional.

In some embodiments, a functional variant of a CRM can be viewed as a CRM which, when substituted in place of a reference CRM in a promoter, substantially retains its activity. For example, a muscle-specific promoter which comprises a functional variant of a given CRM preferably retains at least 80% of its activity, more preferably at least 90% of its activity, more preferably at least 95% of its activity, and yet more preferably 100% of its activity (compared to the reference promoter comprising the unmodified CRM).

Suitably, functional variants of a CRM retain a significant level of sequence identity to a reference CRM. Suitably functional variants comprise a sequence that is at least 70% identical to the reference CRM, more preferably at least 70%, 80%, 90%, 91%, 92%, 93%, 94%, 95%, 96%, 97%, 98% or 99% identical to the reference CRM.

Retention of activity can be assessed by comparing expression of a suitable reporter under the control of the reference promoter with an otherwise identical promoter comprising the substituted CRE under equivalent conditions. Suitable methods for assessing muscle-specific promoter activity are disclosed herein, e.g. in the examples.

Functional variants of a given CRM can, in some embodiments, comprise functional variants of one or more of the CREs present in the reference CRM. For example, functional variants of a given CRM can comprise functional variants of 1, 2, 3, 4, 5, or 6 of the CREs present in the reference CRM.

Functional variants of a given CRM can, in some embodiments, comprise the same combination CREs as a reference CRM, but the CREs can be present in a different order from the reference CRM. It is usually preferred that the CREs are present in the same order as the reference CRM (thus, the functional variant of a CRM suitably comprises the same permutation of the CREs as set out in a reference CRM).

Functional variants of a given CRM can, in some embodiments, comprise one or more additional CREs to those present in a reference CRM. Additional CREs can be provided upstream of the CREs present in the reference CRM, downstream of the CREs present in the reference CRM, and/or between the CREs present in the reference CRM. The additional CREs can be CREs disclosed herein, or they can be other CREs. Generally, it is preferred that a functional variant of a given CRM comprises the same CREs (or functional variants thereof) and does not comprise additional CREs.

Functional variants of a given CRM can comprise one or more additional regulatory elements compared to a reference CRM. For example, they may comprise an inducible or repressible element, an intronic element, a boundary control element, an insulator, a locus control region, a response element, a binding site, a segment of a terminal repeat, a responsive site, a stabilizing element, a de-stabilizing element, de-targeting element, liver de-targeting element and a splicing element, etc., provided that they do not render the CRM substantially non-functional.

Functional variants of a given CRM can comprise additional spacers between adjacent CREs or, if one or more spacers are present in the reference CRM, said one or more spacers can be longer or shorter than in the reference CRM (or absent).

It will be apparent that the CRMs as disclosed herein, or functional variants thereof, can be combined with any suitable promoter elements in order to provide a synthetic muscle-specific promoter according to the present invention.

In some embodiments, the synthetic muscle-specific CRM according to the present invention is operably linked to a promoter element to form a synthetic muscle-specific promoter. The promoter element may be a minimal or proximal promoter. The proximal promoter is preferably a muscle-specific proximal promoter. In some embodiments, the synthetic muscle-specific CRM according to the present invention is operably linked to the promoter element SCP1 (SEQ ID NO: 12). In some embodiments, the synthetic muscle-specific CRM according to the present invention is operably linked to the promoter element CRE0053 (SEQ ID NO: 26).

In a CRM, the regulatory elements are preferably present in the recited order and are preferably adjacent to one another (i.e. without any intervening regulatory elements). The regulatory elements may be contiguous or non-contiguous (i.e. they can be positioned immediately adjacent to one another or they can be separated by a spacer or other sequence). In some embodiments, some or all the regulatory elements may be CREs. The CREs are preferably present in the recited order and are preferably adjacent to one another. The CREs may be contiguous or non-contiguous (i.e. they can be positioned immediately adjacent to one another or they can be separated by a spacer or other sequence). In some preferred embodiments, the regulatory elements, or the functional variants thereof, are provided in the recited order and are adjacent to one another. For example, the synthetic skeletal muscle-specific CRM may comprise CRE0145, DES_MT enhancer 48bp and Liver de-targeting sequence 1, and so forth. In some embodiments, it is preferred that some or all of the CREs are contiguous.

In some embodiments, the synthetic muscle-specific CRM comprises one or more regulatory elements in addition to the regulatory elements recited above. In some embodiments, the one or more additional regulatory elements may be one or more other regulatory elements according to this invention or other regulatory elements. In some embodiments, the one or more additional regulatory elements may be one or more CREs according to this invention or other CREs. In some embodiments, the one or more additional regulatory elements may be one or more promoter elements. In some embodiments, the one or more additional regulatory elements may be one or more de-targeting elements or liver de-targeting elements. In some embodiments, the one or more additional regulatory elements may be one or more introns.

In some embodiments the synthetic muscle-specific CRM comprises a sequence which is at least 70%, 75%, 80%, 85%, 90%, 95%, 96%, 97%, 98% or 99% identical to any one of SEQ ID NO: 9, 20-22.

In some embodiments, the synthetic muscle-specific CRM is active in both skeletal and cardiac muscle. In some embodiments, the synthetic muscle-specific CRM is active in skeletal muscle. In some embodiments, the synthetic muscle-specific CRM is active in cardiac muscle.

CRMs comprising CRE0145 and DES_MT_enhancer_48bp have been found to provide significant muscle-specific enhancer activity in both skeletal and cardiac muscle when combined with a suitable promoter element.

In many instances, shorter promoter sequences are preferred, particularly for use in situations where a vector (e.g. a viral vector such as AAV) has limited capacity. Accordingly, in some embodiments the synthetic muscle-specific CRM has length of 300 or fewer nucleotides, for example 290, 280, 270, 260, 250, 240, 230, 220, 210, 200, 150, 100, 75, 60, 50 or fewer nucleotides. In some embodiments, the synthetic muscle-specific CRM has length of 270 or fewer nucleotides, preferably 220 or fewer nucleotides, more preferably 190 or fewer nucleotides, most preferably 170 or fewer nucleotides.

### Synthetic Muscle-Specific Promoters and Functional Variants Thereof

Various synthetic muscle-specific promoters are disclosed herein. A functional variant of a reference synthetic muscle-specific promoter is a promoter which comprises a sequence which varies from the reference synthetic muscle-specific promoter, but which substantially retains muscle-specific promoter activity. It will be appreciated by the skilled person that it is possible to vary the sequence of a synthetic muscle-specific promoter while retaining its ability to recruit suitable muscle-specific transcription factors (TFs) and to recruit RNA polymerase II to provide muscle-specific expression of an operably linked sequence (e.g. an open reading frame). A functional variant of a synthetic muscle-specific promoter can comprise substitutions, deletions and/or insertions compared to a reference promoter, provided such substitutions, deletions and/or insertions do not render the synthetic muscle-specific promoter substantially non-functional compared to the reference promoter.

Accordingly, in some embodiments, a functional variant of a synthetic muscle-specific promoter can be viewed as a variant which substantially retains the muscle-specific promoter activity of the reference promoter. For example, a functional variant of a synthetic muscle-specific promoter preferably retains at least 70% of the activity of the reference promoter, more preferably at least 80% of its activity, more preferably at least 90%, 91%, 92%, 93%, 94% of its activity, more preferably at least 95%, 96%, 97%, 98% of its activity, and yet more preferably 100% of its activity. In some embodiments, the functional variant of a synthetic muscle-specific promoter retains at least 25%, 50%, 75%, 80%, 85%, 90%, 95% or 100% of the activity of the reference promoter.

Functional variants of a synthetic muscle-specific promoter often retain a significant level of sequence similarity to a reference synthetic muscle-specific promoter. In some embodiments, functional variants comprise a sequence that is at least 70% identical to the reference synthetic muscle-specific promoter, more preferably at least 80%, 90%, 91%, 92%, 93%, 94%, 95%, 96%, 97%, 98% or 99% identical to the reference synthetic muscle-specific promoter.

In one embodiment, the synthetic muscle-specific promoters described herein increases expression, e.g., of a gene product driven by the promoter, in the muscle by at least 10% as compared to a reference level. In one embodiment, the synthetic muscle-specific promoters described herein increases expression, e.g., of a gene product driven by the promoter, in the muscle by at least 5%, 10%, 15%, 20%, 25%, 30%, 35%, 40%, 45%, 50%, 55%, 60%, 65%, 70%, 75%, 80%, 85%, 90%, 95%, 99%, or more as compared to a reference level, or at least 10-fold, 20-fold, 30-fold, 40-fold, 50-fold, 60-fold, 70-fold, 80-fold, 90-fold, 100-fold, 150-fold, 200-fold, 250-fold, 300-fold, 350-fold, 400-fold, 450-fold, 500-fold, or more as compared to a reference level. As used herein, "reference level" refers to the expression level of a product (e.g., a promoter or gene product driven by the promoter) in the muscle driven by a non-synthetic muscle-specific promoter in an otherwise identical sample. One skilled in the art can assess the expression level in of a given product in the muscle using standard techniques, e.g., PCR-based assays or western blotting to measure mRNA or protein levels, respectively.

Activity in a functional variant can be assessed by comparing expression of a suitable reporter under the control of the reference synthetic muscle-specific promoter with the putative functional variant under equivalent conditions. Suitable methods for assessing muscle-specific promoter activity are disclosed herein, e.g. in the examples.

Functional variants of a given synthetic muscle-specific promoter can comprise functional variants of one or more CREs present in the reference synthetic muscle-specific promoter. Functional variants of CREs are discussed above.

Functional variants of a given synthetic muscle-specific promoter can comprise functional variants of the CRM present in the reference synthetic muscle-specific promoter. Functional variants of CRMs are discussed above.

Functional variants of a given synthetic muscle-specific promoter can comprise functional variants of the promoter element, or a different promoter element when compared to the reference synthetic muscle-specific promoter. Functional variants of promoter elements are discussed above.

Functional variants of a given synthetic muscle-specific promoter can comprise the same CREs as a reference synthetic muscle-specific promoter, but the CREs can be present in a different order from the reference synthetic muscle-specific promoter.

Functional variants of a given synthetic muscle-specific promoter can comprise one or more additional CREs to those present in a reference synthetic muscle-specific promoter. Additional CREs can be provided upstream of the CREs present in the reference synthetic muscle-specific promoter, downstream of the CREs present in the reference synthetic muscle-specific promoter, and/or between the CREs present in the reference synthetic muscle-specific promoter. The additional CREs can be CREs disclosed herein, or they can be other CREs.

Functional variants of a given synthetic muscle-specific promoter can comprise additional spacers between adjacent CREs and promoter elements or, if one or more spacers are present in the reference synthetic muscle-specific promoter, said one or more spacers can be longer or shorter than in the reference synthetic muscle-specific promoter (or it can be absent).

It will be apparent that synthetic muscle-specific promoters of the present invention can comprise a CRM of the present invention and additional regulatory elements. For example, they may comprise one or more additional CRMs, an inducible or repressible element, a boundary control element, an insulator, a locus control region, a response element, a binding site, a segment of a terminal repeat, a responsive site, a stabilizing element, a de-stabilizing element, de-targeting element, liver de-targeting element, an intron and a splicing element, etc., provided that they do not render the promoter substantially non-functional.

Preferred synthetic muscle-specific promoters of the present invention exhibit muscle-specific promoter activity which is at least 15%, 20%, 25%, 30%, 40%, 50%, 60%, 70%, 80%, 90%, 100%, 125%, 150%, 175%, 200%, 250%, 300%, 350% or 400% of the activity exhibited by the CBA, CK8, CK7 or RSV promoter in muscle cells. In many cases higher levels of promoter activity is preferred, but this is not always the case; thus, in some cases more moderate levels of expression may be preferred. In some cases, it is desirable to have available a range of promoters of different activity levels to allow the level of expression to by tailored to requirements; the present disclose provides promoters with such a range of activities. Activity of a given synthetic muscle-specific promoter of the present invention compared to CBA, CK8, CK7 or RSV can be assessed by comparing muscle-specific expression of a reporter gene under control of the synthetic muscle-specific promoter with expression of the same reporter under control of the CBA, CK8, CK7 or RSV promoter, when the two promoters are provided in otherwise equivalent expression constructs and under equivalent conditions. Suitable methods to test the activity of muscle-specific promoters can be found e.g. in the examples.

Suitably, a synthetic muscle-specific promoter of the invention may be able to increase expression of a gene (e.g. a therapeutic gene or gene of interest) in the muscle of a subject or in a muscle cell by at least 20%, at least 40%, at least 60%, at least 80%, at least 100%, at least 200%, at least 300%, at least 500%, at least 1000% or more relative to a known muscle-specific promoter, suitably the SPc5-12 promoter (Gene Ther. 2008 Nov; 15(22): 1489-99).

Suitably, synthetic muscle-specific promoters of the present invention exhibit activity in non-muscle cells (e.g. Huh7 and HEK293 cells) which is 50% or less when compared to CMV-IE, preferably 25% or less than CMV-IE, more preferably 10% or less than CMV-IE, and in some cases 5% or less than CMV-IE, or 1% or less than CMV-IE.

In many instances, shorter promoter sequences are preferred, particularly for use in situations where a vector (e.g. a viral vector such as AAV) has limited capacity. Accordingly, in some embodiments the synthetic muscle-specific promoter has length of 400 or fewer nucleotides, for example, 390, 380, 370, 360, 350, 340, 330, 320, 310, 300, 290, 280, 270, 260, 250 or fewer nucleotides. In some embodiments the synthetic muscle-specific promoter has a length of 390 or fewer nucleotides, more preferably 380 or fewer nucleotides, yet more preferably 350 or fewer nucleotides, most preferably 330 or fewer nucleotides. In some embodiments the synthetic muscle-specific promoter has a length of 320 or fewer nucleotides, preferably 310 or fewer nucleotides, more preferably 280 or fewer nucleotides, most preferably 250 or fewer nucleotides.

Particularly preferred synthetic muscle-specific promoters are those that are both short and which exhibit high levels of activity.

In some embodiments, the synthetic muscle-specific promoter comprises a sequence which is at least 70%, 75%, 80%, 85%, 90%, 95%, 96%, 97%, 98% or 99% identical to any one of SEQ ID NOs: 1-4.

It is generally preferred that a promoter according to the present invention which is a functional variant of any one of SEQ ID NOs:1-4 retains at least 25%, 50%, 75%, 80%, 85%, 90%, 95% or 100% of the activity of the reference promoter. Suitably said activity is assessed using one of the examples as described herein, but other methods can be used.

In some embodiments, the synthetic muscle-specific promoter is active in both skeletal and cardiac muscle. In some embodiments, the synthetic muscle-specific promoter is active in skeletal muscle. In some embodiments, the synthetic muscle-specific promoter is active in cardiac muscle.

Synthetic promoters comprising CRE0145 and DES_MT_enhancer_48bp operably linked to SCP1 have been found to provide significant muscle-specific enhancer activity in both skeletal and cardiac muscle.

Synthetic promoters comprising CRE0145 and DES_MT enhancer 48bp operably linked to CRE0053 have been found to provide significant cardiac muscle-specific activity.

In a synthetic muscle-specific promoter, the regulatory elements are preferably present in the recited order and are preferably adjacent to one another (i.e. without any intervening regulatory elements). The regulatory elements may be contiguous or non-contiguous (i.e. they can be positioned immediately adjacent to one another or they can be separated by a spacer or other sequence). In some embodiments, some or all the regulatory elements may be CREs. The CREs are preferably present in the recited order and are preferably adjacent to one another. The CREs may be contiguous or non-contiguous (i.e. they can be positioned immediately adjacent to one another or they can be separated by a spacer or other sequence). In some preferred embodiments, the regulatory elements, or the functional variants thereof, are provided in the recited order and are adjacent to one another. The promoter element lies downstream of the regulatory elements and is typically adjacent to the proximal regulatory element. The promoter element may be contiguous with the adjacent regulatory element, or it can be separated by a spacer. The promoter element lies downstream of the CREs and is typically adjacent to the proximal CRE. The promoter element may be contiguous with the adjacent CRE, or it can be separated by a spacer.

In some embodiments, the promoter element lies downstream of the regulatory elements, and typically it is adjacent to the proximal regulatory element. The promoter element may be contiguous with the adjacent regulatory element, or it can be separated by a spacer.

In some embodiments, the synthetic muscle-specific promoter comprises one or more regulatory elements in addition to the regulatory elements recited above. In some embodiments, the one or more additional regulatory elements may be one or more other regulatory elements according to the present invention or other regulatory elements. In some embodiments, the one or more additional regulatory elements may be one or more other CREs according to the present invention or other CREs. In some embodiments, the one or more additional regulatory elements may be one or more promoter elements. In some embodiments, the one or more additional regulatory elements may be one or more de-targeting elements or liver de-targeting elements. In some embodiments, the one or more additional regulatory elements may be one or more introns.

### Synthetic Muscle-Specific Expression Cassettes

Disclosed herein are also muscle-specific expression cassette. The muscle-specific expression cassette may comprise a synthetic muscle-specific promoter according to any aspect of the present invention operably linked to a sequence encoding an expression product, suitably a gene (e.g. a transgene such as a therapeutic transgene).

In some embodiments, the expression product is a therapeutic expression product. In some preferred embodiments, the therapeutic expression product is suitable for use in treating a disease or condition. In some embodiments, the disease or condition is associated with aberrant gene expression, optionally in the muscle (i.e. muscular disease), optionally in cardiac muscle and/or in skeletal muscle. In some preferred embodiments, therapeutic expression products include those useful in the treatment of disease, suitably muscle diseases.

The term "muscular disease" is, in principle, understood by the skilled person. The term relates to a disease amenable to treatment and/or prevention by administration of an active compound to a muscle, in particular to a muscle cell. In some embodiments, the muscular disease is a skeletal muscle disease. In some embodiments, the muscular disease is a cardiac muscle disease. In some embodiments, the muscular disease is a skeletal and cardiac muscle disease.

Suitable diseases and expression products are detailed below.

Suitably the synthetic muscle-specific expression cassette comprises sequences providing or coding for one or more of, and preferably all of, a ribosomal binding site, a start codon, a stop codon, and a transcription termination sequence. Suitably the expression cassette comprises a nucleic acid encoding a post-transcriptional regulatory element. Suitably the expression cassette comprises a nucleic acid encoding a polyA element.

### Vectors and Viral Particles

Various vector comprising a synthetic muscle-specific promoter, or expression cassette according to the present invention are disclosed herein.

In some embodiments of the invention, the vector is a plasmid. Such a plasmid may include a variety of other functional nucleic acid sequences, such as one or more selectable markers, one or more origins of replication, multiple cloning sites and the like. In some embodiments of the invention, the vector is a viral vector.

In some embodiments of the invention, the vector is an expression vector for expression in eukaryotic cells. Examples of eukaryotic expression vectors include, but are not limited to, pW-LNEO, pSV2CAT, pOG44, pXTI and pSG available from Stratagene; pSVK3, pBPV, pMSG and pSVL available from Amersham Pharmacia Biotech; and pCMVDsRed2-express, pIRES2-DsRed2, pDsRed2-Mito, pCMV-EGFP available from Clontech. Many other vectors are well-known and commercially available. For mammalian adenoviral vectors, the pSV and the pCMV series of vectors are particularly well-known non-limiting examples. There are many well-known yeast expression vectors including, without limitation, yeast integrative plasmids (Yip) and yeast replicative plasmids (Yrp). For plants, the Ti plasmid of agrobacterium is an exemplary expression vector, and plant viruses also provide suitable expression vectors, e.g. tobacco mosaic virus (TMV), potato virus X, and cowpea mosaic virus.

In some preferred embodiments, the vector is a gene therapy vector. Various gene therapy vectors are known in the art, and mention can be made of AAV vectors, adenoviral vectors, retroviral vectors and lentiviral vectors. Where the vector is a gene therapy vector, the vector preferably comprises a nucleic acid sequence operably linked to the synthetic muscle-specific promoter of the invention that encodes a therapeutic product, suitably a therapeutic protein. The therapeutic protein may be a secretable protein. Non-limiting examples of secretable proteins are discussed above, and exemplary secretable therapeutic proteins, include clotting factors, such as factor VIII or factor IX, insulin, erythropoietin, lipoprotein lipase, antibodies or nanobodies, growth factors, cytokines, chemokines, plasma factors, toxic proteins, etc.

In some embodiments of the invention, the vector is a viral vector, such as a retroviral, lentiviral, adenoviral, or adeno-associated viral (AAV) vector. In some preferred embodiments, the vector is an AAV vector. In some preferred embodiments, the AAV has a serotype suitable for muscle transduction. In some embodiments, the AAV is selected from the group consisting of: AAV2, AAV5, AAV6, AAV7, AAV8, AAV9 BNP116, rh10, AAV2.5, AAV2i8, AAVDJ8 and AAV2G9, or derivatives thereof. AAV vectors are preferably used as self-complementary, double-stranded AAV vectors (scAAV) in order to overcome one of the limiting steps in AAV transduction (i.e. single-stranded to double-stranded AAV conversion), although the use of single-stranded AAV vectors (ssAAV) is also encompassed herein. In some embodiments of the invention, the AAV vector is chimeric, meaning it comprises components from at least two AAV serotypes, such as the ITRs of an AAV2 and the capsid protein of an AAV5. AAV9 is known to effectively transduce skeletal muscle and cardiac muscle particularly effectively, and thus AAV9 and derivatives thereof are of particular interest for targeting skeletal and cardiac muscle. AAV1, AAV6, AAV7 and AAV8 are also known to target skeletal muscle, and thus these AAV serotypes and derivatives thereof are also of particular interest for targeting skeletal muscle. AAV1 and AAV8 are also known to target cardiac muscle, and thus these AAV serotypes and derivatives thereof are also of particular interest for targeting cardiac muscle. In some embodiments, the rAAV vector is a AAV3b serotype, including, but not limited to, an AAV3b265D virion, an AAV3b265D549A virion, an AAV3b549A virion, an AAV3bQ263Y virion, or an AAV3bSASTG virion (i.e., a virion comprising a AAV3b capsid comprising Q263A/T265 mutations). In some embodiments, the virion can be rational haploid, or a chimeric or any mutant, such as capsids can be tailored for increased update at a desired location, e.g., the heart. Other capsids can include capsids from any of the known AAV serotypes, including AAV1, AAV3, AAV4, AAV5, AAV7, AAV10, etc. In some embodiments, the vector is AAV9. In some embodiments, the vector is AAVMYO. The AAVMYO capsid has been shown to have high transduction in muscle and is described in Weinmann, J., Weis, S., Sippel, J. et al. Identification of a myotropic AAV by massively parallel in vivo evaluation of barcoded capsid variants. Nat Commun 11, 5432 (2020). https://doi.org/10.1038/s41467-020-19230-w which is incorporated herein by reference. In some embodiments, the vector is a MyoAAV vector. The MyoAAV capsids has been shown to have high transduction in muscle and is described in Mohammadsharif Tabebordbar, Kim A. Lagerborg, Alexandra Stanton, Emily M. King, Simon Ye, Liana Tellez, Allison Krunnfusz, Sahar Tavakoli, Jeffrey J. Widrick, Kathleen A. Messemer, Emily C. Troiano, Behzad Moghadaszadeh, Bryan L. Peacker, Krystynne A. Leacock, Naftali Horwitz, Alan H. Beggs, Amy J. Wagers, Pardis C. Sabeti, Directed evolution of a family of AAV capsid variants enabling potent muscle-directed gene delivery across species, Cell, Volume 184, Issue 19, 2021, Pages 4919-4938.e22, ISSN 0092-8674, https://doi.org/10.1016/j.cell.2021.08.028 which is incorporated herein by reference.

The invention further provides recombinant virions (viral particles) comprising a vector as described above.

### Pharmaceutical Compositions

The synthetic muscle-specific promoter, expression cassette, vectors or virions of the present invention may be formulated in a pharmaceutical composition with one or more pharmaceutically acceptable excipients, i.e., one or more pharmaceutically acceptable carrier substances and/or additives, e.g., buffers, carriers, excipients, stabilisers, etc. The pharmaceutical composition may be provided in the form of a kit. Pharmaceutical compositions and delivery systems appropriate for the AAV vectors and methods and uses of are known in the art.

### Therapeutic and Other Methods and Uses

The synthetic muscle-specific promoter, expression cassette, vector, virion or pharmaceutical composition according to various aspects of the present invention may be for use in the treatment of a disease, preferably a disease associated with aberrant gene expression, optionally in the muscle ( e.g., muscular disease)

In one embodiment, the present invention provides a synthetic muscle-specific promoter, expression cassette, vector, virion or pharmaceutical composition according to various aspects of the present invention for use in the treatment of a skeletal muscle disease. In one embodiment, the present invention also provides a synthetic muscle-specific promoter, expression cassette, vector, virion or pharmaceutical composition according to various aspects of the present invention for use in the treatment of a cardiac muscle disease. In one embodiment, the present invention provides a synthetic muscle-specific promoter, expression cassette, vector, virion or pharmaceutical composition according to various aspects of the present invention for use in the treatment of a skeletal and cardiac muscle disease.

Relevant diseases and expression products are discussed below.

The synthetic muscle-specific promoter, expression cassette, vector, virion according to the various aspects of the present invention may be for use in the manufacture of a pharmaceutical composition for treatment of any condition or disease mentioned herein.

### A cell

The present invention further provides a cell comprising a synthetic muscle-specific promoter, expression cassette, vector, virion according to the various aspects of the invention. Suitably the cell is a eukaryotic cell. The eukaryotic cell can suitably be a fungal cell (e.g. yeast cell), an animal (metazoan) cell (e.g. a mammalian cell), or a plant cell. Alternatively, the cell may be a prokaryotic cell. In some embodiments of the invention, the cell is ex *vivo,* e.g. in cell culture. In other embodiments of the invention the cell may be part of a tissue or multicellular organism.

In a preferred embodiment, the cell is a muscle cell (myocyte), which may be ex *vivo or in vivo.* In a preferred embodiment, the cell is a cardiac muscle cell, which may be ex *vivo or in vivo.* In an alternative preferred embodiment, the cell is a skeletal muscle cell, which may be ex *vivo or in vivo.* The muscle cell may be a primary muscle cell or a cell of a muscle-derived cell line, e.g. an immortalised cell line. The cell may be present within a muscle tissue environment (e.g. within a muscle of an animal) or may be isolated from muscle tissue, e.g. it may be in cell culture. Suitably the cell is a human cell.

The skeletal muscle cells may be from fast twitch or slow twitch muscles. The skeletal muscle cells may be selected from intermediate twitch cells, myocytes, myotubes, myoblasts and satellite cells. The cardiac muscle cells may be selected from ventricular cardiomyocytes, atrial cardiomyocytes, pericytes, cardiac smooth muscle cells, cardiac fibroblasts, or endothelial cells (EC) in the heart, as well as peri-vascular cells and pacemaker cells.

The synthetic muscle-specific promoter, expression cassette, or vector, according to the invention may be inserted into the genome of the cell, or it may be episomal (e.g. present in an episomal vector).

### Method for producing an expression product

Disclosed herein is also a method for producing an expression product, the method comprising providing a synthetic muscle-specific expression cassette according to the present invention (preferably in a vector as set out above) in a cell, preferably a muscle cell, and expressing the expression product, suitably the gene, present in the synthetic muscle-specific expression cassette. The method suitably comprises maintaining said muscle cell under suitable conditions for expression of the expression product, suitably a gene. In culture this may comprise incubating the cell, or tissue comprising the cell, under suitable culture conditions. Suitably, culture conditions may be incubation at 37°C, 5% CO2. The expression may of course be *in vivo*, e.g. in one or more cells in the muscle of a subject. In one embodiment, the muscle cell/s are cardiac muscle cell/s. In one embodiment, the muscle cell/s are skeletal muscle cell/s.

Suitably the method comprises the step of introducing the synthetic muscle-specific expression cassette into the muscle cell. A wide range of methods of transfecting muscle cells are well-known in the art. A preferred method of transfecting muscle cells is transducing the cells with a viral vector comprising the synthetic muscle-specific expression cassette, e.g. an AAV vector.

A wide range of methods of transfecting cells are well known in the art, for example virus mediated transfection such as transfection with viral vectors; chemical-based transfection such as lipofection, transfection with calcium phosphate, cationic polymers of Fugene reagents; non-chemical based transfection such as electroporation; microinjection; agrobacterium-mediated transfer; gene gun; impalefection; hydrostatic pressure; direct DNA uptake; whiskers-mediated transformation; and microprojectile bombardment.

### Method of expressing an expression product

Disclosed herein is also a method of expressing an expression product, suitably a therapeutic transgene in a muscle cell, the method comprising introducing into the muscle cell an expression cassette or vector according to the present invention. Suitably introducing the expression cassette or vector may comprise transfecting the muscle cell with the expression cassette or vector. A wide range of methods of transfecting muscle cells are well-known in the art. A preferred method of transfecting muscle cells is transducing the cells with a viral vector comprising the synthetic muscle-specific expression cassette, e.g. an AAV vector. The muscle cell can be *in vivo* or ex *vivo.* In one embodiment, the muscle cell/s are cardiac muscle cell/s. In one embodiment, the muscle cell/s are skeletal muscle cell/s.

### Method of gene therapy

It will be evident to the skilled person that a synthetic muscle-specific promoter, expression cassette, vector or virion according to various aspects of the invention may be used for gene therapy. Accordingly, the use of such nucleic acid constructs in gene therapy forms part of the present invention.

The expression cassette, vector or virion according to the present invention may be for use in gene therapy in a subject, preferably gene therapy through muscle-specific expression of an expression product, suitably a therapeutic gene. Suitably, the expression cassette, vector or virion according to the present invention may be for use in gene therapy through cardiac muscle-specific expression and/or skeletal muscle-specific expression of an expression product, suitably a therapeutic gene. The therapy may involve treatment of a disease through secretion of an expression product, suitably a therapeutic product, from muscle cells, suitably a disease involving aberrant gene expression in the muscle. Suitable diseases are discussed below.

Disclosed herein is also a method of gene therapy of a subject, preferably a human, in need thereof, the method comprising:
- administering to the subject (suitably introducing into the muscle of the subject) a synthetic muscle-specific expression cassette, vector, virion or pharmaceutical composition of the present invention, which comprises expression product, suitably a gene encoding for a therapeutic expression product. Suitable expression products are detailed below.

In one embodiment, the muscle is cardiac muscle. In one embodiment, the muscle is skeletal muscle.

The method suitably comprises expressing a therapeutic amount of the therapeutic product from the gene in the muscle of said subject. Various conditions and diseases that can be treated and suitable diseases are discussed below.

In some embodiments, the method comprises administering a vector or virion according to the present invention to the subject. Suitably, the vector is a viral gene therapy vector, for example an AAV vector.

In some embodiments, the method comprises administering the viral gene therapy vector systemically. Systemic administration may be enteral (e.g. oral, sublingual, and rectal) or parenteral (e.g. injection). Preferred routes of injection include intravenous, intramuscular, subcutaneous, intra-arterial, intra-articular, intrathecal, and intradermal injections.

In some embodiments, the viral gene therapy vector may be administered concurrently or sequentially with one or more additional therapeutic agents or with one or more saturating agents designed to prevent clearance of the vectors by the reticular endothelial system.

Where the vector is an AAV vector, the dosage of the vector may be from 1×10¹⁰ gc/kg to 1×10¹⁵ gc/kg or more, suitably from 1×10¹² gc/kg to 1×10¹⁴ gc/kg, suitably from 5×10¹² gc/kg to 5×10¹³ gc/kg (gc/kg stands for genome copies per kilogram).

In general, the subject in need thereof will be a mammal, and preferably primate, more preferably a human. Typically, the subject in need thereof will display symptoms characteristic of a disease. The method typically comprises ameliorating the symptoms displayed by the subject in need thereof, by expressing the therapeutic amount of the expression product, suitably a therapeutic expression product.

Gene therapy protocols for therapeutic gene expression in target cells *in vitro* and *in vivo,* are well-known in the art and will not be discussed in detail here. Briefly, they include intramuscular injection, interstitial injection, instillation in airways, application to endothelium, intra-hepatic parenchyme, and intravenous or intra-arterial administration (e.g. intra-hepatic artery, intra-hepatic vein) of plasmid DNA vectors (naked or in liposomes) or viral vectors. Various devices have been developed for enhancing the availability of DNA to the target cell. While a simple approach is to contact the target cell physically with catheters or implantable materials containing the relevant vector, more complex approaches can use jet injection devices and suchlike. Gene transfer into mammalian muscle cells has been performed using both ex *vivo* and *in vivo* procedures. The ex *vivo* approach typically requires harvesting of the muscle cells, *in vitro* transduction with suitable expression vectors, followed by reintroduction of the transduced myocytes into the muscle. *In vivo* gene transfer has been achieved by injecting DNA or viral vectors into the muscle. In some preferred embodiments, the preferred route of administration is intravenous. Intravenous delivery is particularly preferred for gene therapy targeting the muscle. In some preferred embodiments, the preferred route of administration is intracoronary. Intracoronary delivery is particularly preferred for gene therapy targeting the cardiac muscle.

According to some preferred embodiments, the methods set out above may be used for the treatment of a subject with a disease as discussed below, e.g. a muscular dystrophy or congestive heart failure.

### Disease

The disease may be any disease. In preferred embodiments, the disease is a disease which can be alleviated by muscle-specific expression of a suitable expression product.

In some embodiments, the disease is a vascular disease, a muscular dystrophy, a cardiomyopathy, a myotonia, a muscular atrophy, a myoclonus dystonia (affected gene: SGCE), a mitochondrial myopathy, a rhabdomyolysis, a fibromyalgia, and/or a myofascial pain syndrome.

In one embodiment, the disease may be a cardiovascular condition, a heart disease or a heart disorder. In one embodiment, the disease may be heart failure such as congestive heart failure. In one embodiment, the disease may be selected from ischemia, arrhythmia, myocardial infarction (MI), abnormal heart contractility, non-ischemic cardiomyopathy, peripheral arterial occlusive disease, and abnormal Ca²⁺ metabolism, and combinations thereof. In some embodiments, the disease may be selected from the group of: congestive heart failure, cardiomyopathy, myocardial infarction, tissue ischemia, cardiac ischemia, vascular disease, acquired heart disease, congenital heart disease, atherosclerosis, dysfunctional conduction systems, dysfunctional coronary arteries, pulmonary heart hypertension. In some embodiments, the disease may be selected from congestive heart failure, coronary artery disease, myocardial infarction, myocardial ischemia, atherosclerosis, cardiomyopathy, idiopathic cardiomyopathy, cardiac arrhythmias, muscular dystrophy, muscle mass abnormality, muscle degeneration, infective myocarditis, drug- or toxin-induced muscle abnormalities, hypersensitivity myocarditis, an autoimmune endocarditis and congenital heart disease.

In some embodiments, the disease is a cardiomyopathy. In some embodiments, the cardiomyopathy is hypertrophic cardiomyopathy, arrhythmogenic right ventricular dysplasia, dilated cardiomyopathy, restrictive cardiomyopathy, left ventricular noncompaction, Takotsubo cardiomyopathy, myocarditis, eosinophilic myocarditis, and ischemic cardiomyopathy. Preferably, the arrhythmogenic right ventricular dysplasia is ARVD1 (Gene: TGFB3), ARVD2 (Gene: RYR2), ARVD3, ARVD4, ARVD5 (Gene: TMEM43), ARVD6, ARVD7 (Gene: DES), ARVD8 (Gene: DSP), ARVD9 (Gene: PKP2), ARVD10 (Gene: DSG2), ARVD11 (Gene: DSC2), and/or ARVD12 (Gene: JUP). In some embodiments, the disease is hypertrophic cardiomyopathy. Preferably, the hypertrophic cardiomyopathy is CMH1 (Gene: MYH7), CMH2 (Gene: TNNT2), CMH3 (Gene: TPM1), CMH4 (Gene: MYBPC3), CMH5, CMH6 (Gene: PRKAG2), CMH7 (Gene: TNNI3), CMH8 (Gene: MYL3), CMH9 (Gene: TTN), CMH10 (Gene: MYL2), CMH11 (Gene: ACTC1), or CMH12 (Gene: CSRP3).

In some embodiments, the disease is a vascular disease. Vascular disease may be coronary artery disease, peripheral arterial disease, cerebrovascular disease, renal artery stenosis or aortic aneurysm.

In some embodiments, the disease may be cardiomyopathy. The cardiomyopathy may be hypertensive heart disease, heart failure (such as congestive heart failure), pulmonary heart disease, cardiac dysrhythmias, inflammatory heart disease (such as endocarditis, inflammatory cardiomegaly, myocarditis), valvular heart disease, congenital heart disease and rheumatic heart disease.

In some embodiments, the disease is muscular dystrophy. In some embodiments, the muscular dystrophy is Duchenne muscular dystrophy (gene affected: DMD), Becker muscular dystrophy (gene affected: DMD), Limb girdle muscular dystrophy (Subtypes and affected genes: LGMD1A (Gene: TTID), LGMD1B (Gene: LMNA), LGMD1C (Gene: CAV3), LGMD1D (Gene: DNAJB6), LGMD1E (Gene: DES), LGMD1F (Gene: TNP03), LGMD1G (Gene: HNRPDL), LGMD1H, LGMD2A (Gene: CAPN3), LGMD2B (Gene: DYSF), LGMD2C (Gene: SGCG), LGMD2D (Gene: SGCA), LGMD2E (Gene: SGCB), LGMD2F (Gene: SGCD), LGMD2G (Gene: TCAP), LGMD2H (Gene: TRIM32), LGMD2I (Gene: FKRP), LGMD2J (Gene: TTN), LGMD2K (Gene: POMT1), LGMD2L (Gene: AN05), LGMD2M (Gene: FKTN), LGMD2N (Gene: POMT2), LGMD20 (Gene: POMGNT1), LGMD2Q (Gene: PLEC1)), Congenital muscular dystrophy, Distal muscular dystrophy (Subtypes and affected genes: Miyoshi myopathy (Gene: DYSF), Distal myopathy with anterior tibial onset (Gene: DYSF), Welander distal myopathy (Gene: TIA1), Gowers-Laing distal myopathy (Gene: MYH7), Nonaka distal myopathy, hereditary inclusion-body myositis type 1, distal myopathy with vocal cord and pharyngeal weakness, ZASP-related myopathy), Facioscapulohumeral muscular dystrophy (Subtypes and affected genes: Type 1 (Gene: DUX4), Type 2 (Gene: SMCHD1)), Oculopharyngeal muscular dystrophy (affected gene: PABPN1), and/or myotonic dystrophy (Subtypes and affected genes: DM1 (Gene: DMPK) and DM2 (Gene: ZNF9)). In some preferred embodiments, the disease is Limb girdle muscular dystrophy type 2i (LGMD2I; affected gene: FKRP).

In some embodiments, the disease is myotonia. In some embodiments, the myotonia is congenital myotonia (affected gene: CLCN1; subtypes: Type Thomsen, Type Becker) and/or Paramyotonia congenital (affected gene: SCN4A).

In some embodiments, the disease is Duchenne muscular dystrophy (Gene: DMD), a myotubular myopathy (Gene: MTM1), Spinal muscular atrophy (Gene: SMA), Glycogen storage disease type II (Pompe disease, Gene: GAA), or a cardiomyopathy. In some preferred embodiments, the disease is Duchenne muscular dystrophy (gene affected: DMD).

Further exemplary diseases include but are not limited to Acid Maltase Deficiency (AMD), alpha-1 antitrypsin deficiency, Amyotrophic Lateral Sclerosis (ALS), Andersen-Tawil Syndrome, Becker Muscular Dystrophy (BMD), Becker Myotonia Congenita, Bethlem Myopathy, Carnitine Deficiency, Carnitine Palmityl Transferase Deficiency (CPT Deficiency), Central Core Disease (CCD), Centronuclear Myopathy, Charcot-Marie- Tooth Disease (CMT), Congenital Myasthenic Syndromes (CMS), Congenital Myotonic Dystrophy, Cori Disease (Debrancher Enzyme Deficiency), Debrancher Enzyme Deficiency, Dejerine-Sottas Disease (DSD), Dermatomyositis (DM), Endocrine Myopathies, Eulenberg Disease (Paramyotonia Congenita), Forbes Disease (Debrancher Enzyme Deficiency), Friedreich's Ataxia (FA), Glycogenosis Type 10, Glycogenosis Type 11, Glycogenosis Type 2, Glycogenosis Type 3, Glycogenosis Type 5, Glycogenosis Type 7, Glycogenosis Type 9, Gowers-Laing Distal Myopathy, Hauptmann-Thanheuser MD (Emery- Dreifuss Muscular Dystrophy), Hereditary Inclusion-Body Myositis, Hereditary Motor and Sensory Neuropathy (Charcot-Marie-Tooth Disease), Hyperthyroid Myopathy, Hypothyroid Myopathy, Inclusion-Body Myositis (IBM), Inherited Myopathies, Integrin-Deficient Congenital Muscular Dystrophy, Lactate Dehydrogenase Deficiency, Lambert-Eaton Myasthenic Syndrome (LEMS), McArdle Disease (Phosphorylase Deficiency), Metabolic Diseases of Muscle, Mitochondrial Myopathy, Miyoshi Distal Myopathy, Motor Neurone Disease, Muscle-Eye-Brain Disease, Myasthenia Gravis (MG), Myoadenylate Deaminase Deficiency, Myofibrillar Myopathy, Myophosphorylase Deficiency, Myotonia Congenita (MC), Myotonic Muscular Dystrophy (MMD), Myotubular Myopathy (MTM or MM), Nemaline Myopathy, Nonaka Distal Myopathy, Oculopharyngeal Muscular Dystrophy (OPMD), Paramyotonia Congenita, Pearson Syndrome, Periodic Paralysis, Peroneal Muscular Atrophy (Charcot-Marie-Tooth Disease), Phosphofructokinase Deficiency, Phosphogly cerate Kinase Deficiency, Phosphogly cerate Mutase Deficiency, Phosphorylase Deficiency, Phosphorylase Deficiency, Polymyositis (PM), Pompe Disease (Acid Maltase Deficiency), Progressive External Ophthalmoplegia (PEO), Rod Body Disease (Nemaline Myopathy), Spinal Muscular Atrophy (SMA), Spinal-Bulbar Muscular Atrophy (SBMA), Steinert Disease (Myotonic Muscular Dystrophy), Tarui Disease (Phosphofructokinase Deficiency), Thomsen Disease (Myotonia Congenita), Ullrich Congenital Muscular Dystrophy, Walker-Warburg Syndrome (Congenital Muscular Dystrophy), Welander Distal Myopathy, and ZASP-Related Myopathy.

In some preferred embodiments, the disease is a cardiac muscle disease. In some preferred embodiments, the disease is congestive heart failure. In some preferred embodiments, the disease is congenital heart failure.

The promoters according to the present invention are particularly useful in diseases which require expression of a large expression product (e.g. a transgene which is large in size) as they are small in size (e.g. less than 390bp). The promoters according to the present invention are particularly useful in diseases in which there is need to reduce liver expression of the expression product. The promoters according to the present invention are particularly useful in diseases which require expression of a large expression product and there is need to reduce liver expression of the expression product.

### Expression product

The expression product may be any product, expression of which is desired. The expression product may be a gene. The expression product may be a gene encoding a desired gene expression product (e.g. a product of interest) such as a polypeptide (protein) or RNA. The expression product may be a transgene. The expression product may be a protein or a polypeptide. The expression product may be a nucleic acid sequence. The expression product may be a therapeutic expression product.

In some embodiments the expression product, suitably a gene encodes a non-disease mediating variant, e.g. a wildtype variant of at least one human gene selected from the group consisting of DMD GALGT2, SMA, GAA, MTM1, TTID, LMNA, CAV3, DNAJB6, DES, TNP03, HNRPDL, CAPN3, DYSF, SGCG, SGCA, SGCB, SGCD, TCAP, TRIM32, FKRP, TTN, POMT1, AN05, FKTN, POMT2, PFEC1, DYSF, TIA1, MYH7, DUX4, SMCHD, PABPN1, DMPK, ZNF9, CFCN1, SCN4A, MYH7, TNNT2, TPM1, MYBPC3, PRKAG2, TNNI3, MYF3, TTN, MYF2, ACTC1, CSRP3, TGFB3, RYR2, TMEM43, DES, DSP, PKP2, DSG2, DSC2, JUP, and HYPP. In some preferred embodiments, the gene is DMD.

In some embodiments, the expression products include dystrophins (including micro-dystrophins), beta 1,4-n-acetylgalactosamine galactosyltransferase (GALGT2), carbamoyl synthetase I, alpha-1 antitrypsin, ornithine transcarbamylase, arginosuccinate synthetase, arginosuccinate lyase, arginase, fumarylacetacetate hydrolase, phenylalanine hydroxylase, glucose-6-phosphatase, porphobilinogen deaminase, cystathione beta-synthase, branched chain ketoacid decarboxylase, albumin, isovaleryl-coA dehydrogenase, propionyl CoA carboxylase, methyl malonyl CoA mutase, glutaryl CoA dehydrogenase, insulin, beta-glucosidase, pyruvate carboxylate, hepatic phosphorylase, phosphorylase kinase, glycine decarboxylase, H-protein, T-protein, and a cystic fibrosis transmembrane regulator (CFTR).

In some embodiments, the expression product may be the dystrophin protein. Mutations in the DMD gene which impair the function of the dystrophin protein lead to Becker muscular dystrophy or Duchenne muscular dystrophy which are X linked recessive muscular dystrophy disorders characterised by muscle weakness. The DMD gene is the largest known gene in human (approximately 2.4 million base pairs). Therefore, the full-length DMD gene is too large to be packaged into some viral vectors with limited capacity (payload) such as AAV vectors. Shorter versions of the DMD gene (called mini-dystrophins) are being used in order to combat this problem. Nonetheless, even the mini-dystrophins are fairly large (e.g. 3.5 - 4 kB) which still makes AAV packaging difficult. Therefore, a synthetic muscle-specific promoter of short length (e.g. less than 400 nucleotides in length, less than 350 nucleotides in length, preferably less than 300 nucleotides in length, yet more preferably less than 290 nucleotides in length, most preferably less than 280, 270, 260, 250, 240, 230, 220, 210, 200, 150, 100, 75, 70, 68 in length) may be particularly preferred in an expression cassette wherein the sequence encoding the expression product is the DMD gene or a mini version of the DMD gene (mini-dystrophin).

In some preferred embodiment, the expression product is a small version of utrophin (e.g. mini-utrophin or micro-utrophin). In some preferred embodiment, the expression product is a small version of dystrophin (e.g. mini-dystrophin or micro-distrophin). In some embodiments, the expression product may be the miniaturized utrophin disclosed in Song Y, Morales L, Malik AS, Mead AF, Greer CD, Mitchell MA, Petrov MT, Su LT, Choi ME, Rosenblum ST, Lu X, VanBelzen DJ, Krishnankutty RK, Balzer FJ, Loro E, French R, Propert KJ, Zhou S, Kozyak BW, Nghiem PP, Khurana TS, Kornegay JN, Stedman HH. Non-immunogenic utrophin gene therapy for the treatment of muscular dystrophy animal models. Nat Med. 2019 Oct;25(10):1505-1511. doi: 10.1038/s41591-019-0594-0. Epub 2019 Oct 7. PMID: 31591596; PMCID: PMC7274039, and in particular extended data figure 1, which is incorporated herein by reference. In some embodiments, the expression product may be a micro-Dystrophin disclosed in Duan D. Micro-Dystrophin Gene Therapy Goes Systemic in Duchenne Muscular Dystrophy Patients. Hum Gene Ther. 2018 Jul;29(7):733-736. doi: 10.1089/hum.2018.012. Epub 2018 Apr 5. PMID: 29463117; PMCID: PMC6066190 which is incorporated herein by reference. In some embodiments, the expression product may be a micro-utrophin disclosed in Duan D. Micro-utrophin Therapy for Duchenne Muscular Dystrophy. Mol Ther. 2019 Nov 6;27(11):1872-1874. doi: 10.1016/j.ymthe.2019.10.011. Epub 2019 Oct 22. PMID: 31653398; PMCID: PMC6838911, and in particular figure1, which is incorporated herein by reference. In some embodiments, the expression product may be a microutrophin disclosed in Starikova, A.V., Skopenkova, V.V., Polikarpova, A.V. et al. Therapeutic potential of highly functional codon-optimized microutrophin for muscle-specific expression. Sci Rep 12, 848 (2022). https://doi.org/10.1038/s41598-022-04892-x, and in particular figure 1, which is incorporated herein by reference. In some embodiments, the expression product may be a micro-utrophin disclosed in Kennedy TL, Guiraud S, Edwards B, Squire S, Moir L, Babbs A, Odom G, Golebiowski D, Schneider J, Chamberlain JS, Davies KE. Micro-utrophin Improves Cardiac and Skeletal Muscle Function of Severely Affected D2/mdx Mice. Mol Ther Methods Clin Dev. 2018 Oct 16;11:92-105. doi: 10.1016/j.omtm.2018.10.005. PMID: 30417024; PMCID: PMC6216100 which is incorporated herein by reference. In some embodiments, the expression product may be a microutrophin disclosed in Banks GB, Chamberlain JS, Odom GL. Microutrophin expression in dystrophic mice displays myofiber type differences in therapeutic effects. PLoS Genet. 2020 Nov 11;16(11):e1009179. doi: 10.1371/journal.pgen. 1009179. PMID: 33175853; PMCID: PMC7682874, and in particular figure 1, which is incorporated herein by reference. In some embodiments, the expression product may be a micro-dystrophin disclosed in Howard ZM, Dorn LE, Lowe J, Gertzen MD, Ciccone P, Rastogi N, Odom GL, Accornero F, Chamberlain JS, Rafael-Fortney JA. Micro-dystrophin gene therapy prevents heart failure in an improved Duchenne muscular dystrophy cardiomyopathy mouse model. JCI Insight. 2021 Apr 8;6(7):e146511. doi: 10.1172/jci.insight.146511. PMID: 33651713; PMCID: PMC8119181, and in particular the methods, which is incorporated herein by reference. In some embodiments, the expression product may be a micro-dystrophin disclosed in Mendell JR, Sahenk Z, Lehman K, et al. Assessment of Systemic Delivery of rAAVrh74.MHCK7.micro-dystrophin in Children With Duchenne Muscular Dystrophy: A Nonrandomized Controlled Trial. JAMA Neurol. 2020;77(9):1122-1131. doi:10.1001/jamaneurol.2020.1484, and in particular the introduction, which is incorporated herein by reference. In some embodiments, the expression product may be a microdystrophin as dislosed in https://www.clinicaltrials.gov/ct2/show/NCT03368742, which is incorporated herein by reference.

Still other expression products include enzymes useful in enzyme replacement therapy, and which are useful in a variety of conditions resulting from deficient activity of an enzyme. For example, enzymes containing mannose-6-phosphate may be utilized in therapies for lysosomal storage diseases (e.g., a suitable gene includes that encoding β-glucuronidase (GUSB)).

In some embodiments, exemplary polypeptide expression products include neuroprotective polypeptides and anti-angiogenic polypeptides. Suitable polypeptides include, but are not limited to, glial derived neurotrophic factor (GDNF), fibroblast growth factor 2 (FGF-2), nurturin, ciliary neurotrophic factor (CNTF), nerve growth factor (NGF; e.g., nerve growth factor-. Beta.), brain derived neurotrophic factor (BDNF), neurotrophin-3 (NT-3), neurotrophin-4 (NT-4), neurotrophin-6 (NT-6), epidermal growth factor (EGF), pigment epithelium derived factor (PEDF), a Wnt polypeptide, soluble Fit- 1 , angiostatin, endostatin, VEGF, an anti-VEGF antibody, a soluble VEGFR, Factor VIII (FVIII), Factor IX (FIX), and a member of the hedgehog family (sonic hedgehog, Indian hedgehog, and desert hedgehog, etc.).

In some embodiments, the expression products include hormones and growth and differentiation factors including, without limitation, insulin, glucagon, growth hormone (GH), parathyroid hormone (PTH), growth hormone releasing factor (GRF), follicle stimulating hormone (FSH), luteinizing hormone (LH), human chorionic gonadotropin (hCG), vascular endothelial growth factor (VEGF), angiopoietins, angiostatin, granulocyte colony stimulating factor (GCSF), erythropoietin (EPO), connective tissue growth factor (CTGF), basic fibroblast growth factor (bFGF), acidic fibroblast growth factor (aFGF), epidermal growth factor (EGF), platelet- derived growth factor (PDGF), insulin growth factors I and II (IGF-I and IGF-II), any one of the transforming growth factor alpha superfamily, including TGFa., activins, inhibins, or any of the bone morphogenic proteins (BMP) BMPs 1-15, any one of the heregluin/neuregulin/ARIA/neu differentiation factor (NDF) family of growth factors, nerve growth factor (NGF), brain-derived neurotrophic factor (BDNF), neurotrophins NT-3 and NT-4/5, ciliary neurotrophic factor (CNTF), glial cell line derived neurotrophic factor (GDNF), neurturin, agrin, any one of the family of semaphorins/collapsins, netrin-1 and netrin-2, hepatocyte growth factor (HGF), ephrins, noggin, sonic hedgehog and tyrosine hydroxylase.

In some embodiments, the expression products include proteins that regulate the immune system including, without limitation, cytokines and lymphokines such as thrombopoietin (TPO), interleukins (IL) IL-1 through IL-25 (including IL-2, IL-4, IL-12 and IL-18), monocyte chemoattractant protein, leukemia inhibitory factor, granulocyte-macrophage colony stimulating factor, Fas ligand, tumor necrosis factors alpha and beta., interferons (alpha, beta, and gamma), stem cell factor, flk-2/flt3 ligand. Gene products produced by the immune system are also useful in the present invention. In some embodiments, the expression products include immunoglobulins IgG, IgM, IgA, IgD and IgE, chimeric immunoglobulins, humanized antibodies, single chain antibodies, T cell receptors, chimeric T cell receptors, single chain T cell receptors, class I and class II MHC molecules, as well as engineered immunoglobulins and MHC molecules. In some embodiments, the expression products also include complement regulatory proteins such as complement regulatory proteins, membrane cofactor protein (MCP), decay accelerating factor (DAF), CR1, CF2 and CD59.

In some embodiments, the expression products include any one of the receptors for the hormones, growth factors, cytokines, lymphokines, regulatory proteins and immune system proteins. Useful heterologous nucleic acid sequences also include receptors for cholesterol regulation and/or lipid modulation, including the low-density lipoprotein (LDL) receptor, high density lipoprotein (HDL) receptor, the very low-density lipoprotein (VLDL) receptor, and scavenger receptors. The invention also encompasses the use of gene products such as members of the steroid hormone receptor superfamily including glucocorticoid receptors and estrogen receptors, Vitamin D receptors and other nuclear receptors. In addition, useful gene products include transcription factors such as jun, fos, max, mad, serum response factor (SRF), AP-1, AP-2, myb, MyoD and myogenin, ETS-box containing proteins, TFE3, E2F, ATF1, ATF2, ATF3, ATF4, ZF5, NFAT, CREB, HNF-4, C/EBP, SP1, CCAAT-box binding proteins, interferon regulation factor (IRF-1), Wilms tumor protein, ETS-binding protein, STAT, GATA-box binding proteins, e.g., GATA-3, and the forkhead family of winged helix proteins.

In some embodiments, the expression products include those used for treatment of hemophilia, including hemophilia B (including Factor IX) and hemophilia A (including Factor VIII and its variants, such as the light chain and heavy chain of the heterodimer and the B-deleted domain; U.S. Pat. No. 6,200,560 and U.S. Pat. No. 6,221,349).

In some embodiments, the expression product may be a modulator of phosphatase activity, e.g., type 1 phosphatase activity. The modulator may be a protein that inhibits phosphatase activity, e.g., type 1 phosphatase activity. The modulator may be a nucleic acid that increases expression of an endogenous nucleic acid that encodes a protein that inhibits phosphatase activity such as a transcription factor. The modulator may be a regulatory sequence that integrates in or near the endogenous nucleic acid that encodes a protein that inhibits phosphatase activity. The modulator may be a nucleic acid that can provide a nucleic acid modulator of gene expression such as a siRNA.

In some embodiments, the expression product may be inhibitor of protein phosphate 1 (PP1) e.g., a I-1 polypeptide. The phosphatase inhibitor-1 (or "I-1") protein is an endogenous inhibitor of type 1 phosphatase. Increasing I-1 levels or activity can restore β-adrenergic responsiveness in failing human cardiomyocytes. Suitably, the I-1 protein may be constitutively active such as a I-1 protein where threonine 35 is replaced with glutamic acid instead of aspartic acid. The expression product may be any one or more of the inhibitors selected from: phosphatase inhibitor 2 (PP2); okadaic acid or caliculin; and nippl which is an endogenous nuclear inhibitor of protein phosphatase 1.

In some embodiments, the expression product may be any protein that modulates cardiac activity such as a phosphatase type 1 inhibitor, e.g., I-1 or a sacroplasmic reticulum Ca2+ ATPase (SERCA), e.g., SERCA1 (e.g., 1a or 1b), SERCA2 (e.g., 2a or 2b), or SERCA3.

In some embodiments, the expression product may be a nucleic acid sequence encoding a mutant form of phosphatase inhibitor-1 protein, wherein the mutant form comprises at least one amino acid at a position that is a PKC-α phosphorylation site in the wild type, wherein the at least one amino acid is constitutively unphosphorylated or mimics an unphosphorylated state in the mutant form. The expression product may be adenylyl cyclase 6 (AC6, also referred to as adnenylyl cyclase VI), S100A1, β-adrenergic receptor kinase-ct (βARKct), sarco/endoplasmic reticulum (SR) Ca -ATPase (SERCA2a), IL-18, VEGF, VEGF activators, urocortins, and B-cell lymphoma 2 (Bcl2)-associated anthanogene-3 (BAG3).

In some embodiments, the expression product may be an inhibitor of a cytokine such as an IL-18 inhibitor. The expression product may encode a beta-adrenergic signalling protein (beta-ASPs) (including beta-adrenergic receptors (beta-Ars), G-protein receptor kinase inhibitors (GRK inhibitors) and adenylylcyclases (Acs)) to enhance cardiac function.

In some embodiments, the expression product may be an angiogenic protein. Angiogenic proteins promote development and differentiation of blood vessels. Examples of angiogenic proteins include members of the fibroblast growth factor (FGF) family such as aFGF (FGF-1), bFGF (FGF-2), FGF-4 (also known as "hst/KS3"), FGF-5 and FGF-6, the vascular endothelial growth factor (VEGF) family, the platelet-derived growth factor (PDGF) family, the insulin-like growth factor (IGF) family, and others.

In some embodiments, the expression products include non-naturally occurring polypeptides, such as chimeric or hybrid polypeptides having a non-naturally occurring amino acid sequence containing insertions, deletions or amino acid substitutions.

Further suitable expression products include micro RNA (miRNA), interfering RNA, antisense RNA, ribozymes, and aptamers.

In some preferred embodiments, the expression product is an inhibitor of protein phosphate 1 (PP1).

In some embodiments of the invention, the expression product is useful for gene editing, e.g. a gene encoding a site-specific nuclease, such as a meganuclease, zinc finger nuclease (ZFN), transcription activator-like effector-based nuclease (TALEN), or the clustered regularly interspaced short palindromic repeats system (CRISPR-Cas). Suitably the site-specific nuclease is adapted to edit a desired target genomic locus by making a cut (typically a site-specific double-strand break) which is then repaired via non-homologous end-joining (NHEJ) or homology dependent repair (HDR), resulting in a desired edit. The edit can be the partial or complete repair of a gene that is dysfunctional, or the knock-down or knock-out of a functional gene. Alternatively, the edit can be via base editing or prime editing, using suitable systems which are known in the art.

The expression product may be a gene. The gene typically encodes a desired gene expression product such as a polypeptide (protein) or RNA. The gene may be a full-length cDNA or genomic DNA sequence, or any fragment, subunit or mutant thereof that has at least some desired biological activity.

Where the gene encodes a protein, it can be essentially any type of protein. By way of non-limiting example, the protein can be an enzyme, an antibody or antibody fragment (e.g. a monoclonal antibody), a viral protein (e.g. REP-CAP, REV, VSV-G, or RD114), a therapeutic protein, or a toxic protein (e.g. Caspase 3, 8 or 9).

In some preferred embodiments, the gene encodes a therapeutic expression product, preferably a therapeutic polypeptide suitable for use in treating a disease or condition associated with aberrant gene expression, optionally in the muscle, optionally in cardiac muscle and/or in skeletal muscle.

The expression product may be a therapeutic expression product. The therapeutic expression product may be useful in the treatment of a cardiovascular condition or heart disease and disorders such as heart failure or CHF.

The therapeutic expression product may be any protein that modulates cardiac activity such as a phosphatase type 1 inhibitor, e.g., I-1 or a sacroplasmic reticulum Ca2+ ATPase (SERCA), e.g., SERCA1 (e.g., 1a or 1b), SERCA2 (e.g., 2a or 2b), or SERCA3.

The therapeutic expression product may be a modulator of phosphatase activity, e.g., type 1 phosphatase activity. The modulator may be a protein that inhibits phosphatase activity, e.g., type 1 phosphatase activity. The modulator may be a nucleic acid that increases expression of an endogenous nucleic acid that encodes a protein that inhibits phosphatase activity such as a transcription factor. The modulator may be a regulatory sequence that integrates in or near the endogenous nucleic acid that encodes a protein that inhibits phosphatase activity. The modulator may be a nucleic acid that can provide a nucleic acid modulator of gene expression such as a siRNA.

The therapeutic expression product may be inhibitor of protein phosphate 1 (PP1) e.g., a I-1 polypeptide. The phosphatase inhibitor-1 (or "I-1") protein is an endogenous inhibitor of type 1 phosphatase. Increasing I-1 levels or activity can restore β-adrenergic responsiveness in failing human cardiomyocytes. Suitably, the I-1 protein may be constitutively active such as a I-1 protein where threonine 35 is replaced with glutamic acid instead of aspartic acid. The therapeutic expression product may be any one or more of the inhibitors selected from: phosphatase inhibitor 2 (PP2); okadaic acid or caliculin; and nippl which is an endogenous nuclear inhibitor of protein phosphatase 1. In some preferred embodiments, the expression cassette comprises a muscle-specific promoter operably linked to an inhibitor of protein phosphate 1 (PP1). Type 1 phosphatases include, but are not limited to, PP1cα, PP1cβ, PP1cδ and PP1cy.

The therapeutic expression product may be nucleic acid sequence encoding a mutant form of phosphatase inhibitor-1 protein, wherein the mutant form comprises at least one amino acid at a position that is a PKC-α phosphorylation site in the wild type, wherein the at least one amino acid is constitutively unphosphorylated or mimics an unphosphorylated state in the mutant form. The therapeutic expression product may be adenylyl cyclase 6 (AC6, also referred to as adnenylyl cyclase VI), S100A1, β-adrenergic receptor kinase-ct (βARKct), sarco/endoplasmic reticulum (SR) Ca -ATPase (SERCA2a), IL-18, VEGF, VEGF activators, urocortins, and B-cell lymphoma 2 (Bcl2)-associated anthanogene-3 (BAG3).

The therapeutic expression product may be an inhibitor of a cytokine such as an IL-18 inhibitor. The therapeutic expression product may encode a beta-adrenergic signalling protein (beta-ASPs) (including beta-adrenergic receptors (beta-Ars), G-protein receptor kinase inhibitors (GRK inhibitors) and adenylylcyclases (Acs)) to enhance cardiac function.

The therapeutic expression product may be an angiogenic protein. Angiogenic proteins promote development and differentiation of blood vessels. Examples of angiogenic proteins include members of the fibroblast growth factor (FGF) family such as aFGF (FGF-1), bFGF (FGF-2), FGF-4 (also known as "hst/KS3"), FGF-5 and FGF-6, the vascular endothelial growth factor (VEGF) family, the platelet-derived growth factor (PDGF) family, the insulin-like growth factor (IGF) family, and others.

### Definitions and General Points

While the making and using of various embodiments of the present invention are discussed in detail below, it should be appreciated that the present invention provides many applicable inventive concepts that can be embodied in a wide variety of specific contexts. The specific embodiments discussed herein are merely illustrative of specific ways to make and use the invention and do not delimit the scope of the invention.

The discussion of the background to the invention herein is included to explain the context of the invention. This is not to be taken as an admission that any of the material referred to was published, known, or part of the common general knowledge in any country as of the priority date of any of the claims.

Throughout this disclosure, various publications, patents and published patent specifications are referenced by an identifying citation. All documents cited in the present specification are hereby incorporated by reference in their entirety. In particular, the teachings or sections of such documents herein specifically referred to are incorporated by reference.

The practice of the present invention will employ, unless otherwise indicated, conventional techniques of cell biology, cell culture, molecular biology, transgenic biology, microbiology, recombinant DNA, and immunology, which are within the skill of the art. Such techniques are explained fully in the literature. See, for example, Current Protocols in Molecular Biology (Ausubel, 2000, Wiley and son Inc, Library of Congress, USA); Molecular Cloning: A Laboratory Manual, Third Edition, (Sambrook et al, 2001, Cold Spring Harbor, New York: Cold Spring Harbor Laboratory Press); Oligonucleotide Synthesis (M. J. Gait ed., 1984); U.S. Pat. No. 4,683,195; Nucleic Acid Hybridization (Harries and Higgins eds. 1984); Transcription and Translation (Hames and Higgins eds. 1984); Culture of Animal Cells (Freshney, Alan R. Liss, Inc., 1987); Immobilized Cells and Enzymes (IRL Press, 1986); Perbal, A Practical Guide to Molecular Cloning (1984); the series, Methods in Enzymology (Abelson and Simon, eds. -in-chief, Academic Press, Inc., New York), specifically, Vols.154 and 155 (Wu et al. eds.) and Vol. 185, "Gene Expression Technology" (Goeddel, ed.); Gene Transfer Vectors For Mammalian Cells (Miller and Calos eds., 1987, Cold Spring Harbor Laboratory); Immunochemical Methods in Cell and Molecular Biology (Mayer and Walker, eds., Academic Press, London, 1987); Handbook of Experimental Immunology, Vols. I-IV (Weir and Blackwell, eds., 1986); and Manipulating the Mouse Embryo, (Cold Spring Harbor Laboratory Press, Cold Spring Harbor, N.Y., 1986).

To facilitate the understanding of this invention, a number of terms are defined or explained below. Terms used herein have meanings as commonly understood by a person of ordinary skill in the areas relevant to the present invention. Terms such as "a", "an" and "the" are not intended to refer to only a singular entity, but include the general class of which a specific example may be used for illustration. The terminology herein is used to describe specific embodiments of the invention, but their usage does not delimit the invention, except as outlined in the claims.

The term "muscle" is well understood by the skilled person. Preferably, the muscle is a skeletal muscle (including the diaphragm) or a heart muscle. Preferably, the muscle is a muscle of a vertebrate, more preferably of a mammal, even more preferably of a human subject. Preferably, the muscle is a striated muscle. The synthetic promoters of the present invention can be active in skeletal muscle and/or cardiac muscle.

The term "muscle cell" or "myocyte" relates to cells which are found in muscles (muscle tissue) or which are derived from muscle tissue. Muscle cells can be primary cells or a cell line (such as C2C12 or H2K cells (skeletal muscle cell lines) or H9C2 cells (cardiac cell line)). The muscle cells can be in *in vivo* (e.g. in muscle tissue) or *in vitro* (e.g. in cell culture). Myocytes as found in muscle tissue are typically long, tubular cells that develop from myoblasts to form muscles in a process known as myogenesis. The terms muscle cells or myocytes as used herein include myocytes from skeletal muscle and from cardiac muscle (cardiomyocytes). The synthetic promoters of the present invention can be active in skeletal muscle cells and/or cardiac muscle cells.

The term "cis-regulatory element" or "CRE", is a term well-known to the skilled person, and means a nucleic acid sequence such as an enhancer, promoter, insulator, or silencer, that can regulate or modulate the transcription of a neighbouring gene (i.e. in cis). CREs are found in the vicinity of the genes that they regulate. CREs typically regulate gene transcription by binding to TFs, i.e. they include TFBS. A single TF may bind to many CREs, and hence control the expression of many genes (pleiotropy). CREs are usually, but not always, located upstream of the transcription start site (TSS) of the gene that they regulate. "Enhancers" in the present context are CREs that enhance (i.e. upregulate) the transcription of genes that they are operably associated with, and can be found upstream, downstream, and even within the introns of the gene that they regulate. Multiple enhancers can act in a coordinated fashion to regulate transcription of one gene. "Silencers" in this context relates to CREs that bind TFs called repressors, which act to prevent or downregulate transcription of a gene. The term "silencer" can also refer to a region in the 3' untranslated region of messenger RNA, that bind proteins which suppress translation of that mRNA molecule, but this usage is distinct from its use in describing a CRE. Generally, the CREs of the present invention are muscle-specific, cardiac muscle-specific, or skeletal muscle-specific enhancer elements (often referred to as muscle-specific, cardiac muscle-specific, or skeletal muscle-specific CREs, or muscle-specific, cardiac muscle-specific, or skeletal muscle-specific CRE enhancers, or suchlike). In some embodiments, CRE0145 (SEQ ID NO: 10) and DES_MT_enhancer_48bp (SEQ ID NO: 11) are muscle-specific, cardiac muscle-specific, or skeletal muscle-specific enhancer elements. In the present context, it is preferred that the CRE is located 2500 nucleotides or less from the transcription start site (TSS), more preferably 2000 nucleotides or less from the TSS, more preferably 1500 nucleotides or less from the TSS, and suitably 1000, 750, 500, 250, 200, 150, or 100 nucleotides or less from the TSS. CREs of the present invention are preferably comparatively short in length, preferably 500 nucleotides or less in length, for example they may be 400, 300, 200, 175, 150, 90, 80, 70, 60 or 50 nucleotides or less in length. The CREs of the present invention are typically provided in combination with an operably linked promoter element, which can be a minimal promoter or proximal promoter; the CREs of the present invention enhance muscle-specific, cardiac muscle-specific, or skeletal muscle-specific activity of the promoter element. In any of the combinations of CREs, or functional variants thereof, disclosed herein, some or all of the recited CREs and promoter elements may suitably be positioned adjacent to one other in the promoter (i.e. without any intervening CREs or other regulatory elements). The CREs may be contiguous or non-contiguous (i.e. they can be positioned immediately adjacent to one another or they can be separated by a spacer or other sequence). The CREs may be in any order. In some preferred embodiments, the CREs, or functional variants thereof, are provided in the recited order and are adjacent to one another. For example, the synthetic muscle-specific synthetic promoter may comprise CRE0145 immediately upstream of DES_MT_enhancer_48bp, and so forth. In some embodiments it is preferred that some or all of the CREs are contiguous.

The term "cis-regulatory module" or "CRM" means a functional regulatory nucleic acid module, which usually comprises two or more CREs; in the present invention the CREs are typically muscle-specific, cardiac muscle-specific, or skeletal muscle-specific enhancers and thus the CRM is a synthetic muscle-specific, cardiac muscle-specific, or skeletal muscle-specific regulatory nucleic acid. Thus, in the present application a CRM typically comprises a plurality of muscle-specific, cardiac muscle-specific, or skeletal muscle-specific CREs. Typically, the multiple CREs within the CRM act together (e.g. additively or synergistically) to enhance the transcription of a gene that a synthetic promoter comprising the CRM is operably associated with. There is considerable scope to shuffle (i.e. reorder), invert (i.e. reverse orientation), and alter spacing of CREs within a CRM. Accordingly, functional variants of CRMs of the present invention include, inter alia, variants of the referenced CRMs wherein CREs within them have been shuffled and/or inverted, and/or the spacing between CREs has been altered.

As used herein, the phrase "promoter" refers to a region of DNA that generally is located upstream of a nucleic acid sequence to be transcribed that is needed for transcription to occur, i.e. which initiates transcription. Promoters permit the proper activation or repression of transcription of a coding sequence under their control. A promoter typically contains specific sequences that are recognized and bound by plurality of TFs. TFs bind to the promoter sequences and result in the recruitment of RNA polymerase, an enzyme that synthesizes RNA from the coding region of the gene. Many diverse promoters are known in the art.

In some cases, the term "promoter" or "composite promoter" is used herein to refer to a combination of a promoter and additional regulatory elements. In some cases, the additional regulatory elements may be located immediately downstream of the transcription start site (TSS), for example an intron. Such sequences downstream of the TSS can contribute to regulation of expression at the transcriptional and/or translational stages. In some cases, the additional regulatory elements may be located in between CREs and promoter elements, for example a de-targeting element or a liver de-targeting element. Such sequences located in between CREs and promoter elements may contribute to lower expression in specific tissue or cell types, for example in the liver or in liver cells.

The term "synthetic promoter" as used herein relates to a promoter that does not occur in nature. In the present context it typically comprises a CRE and/or CRM of the present invention operably linked to a promoter element, such as minimal (or core) promoter or muscle-specific, cardiac muscle-specific, or skeletal muscle-specific proximal promoter. The CREs and/or CRMs of the present invention serve to enhance muscle-specific, cardiac muscle-specific, or skeletal muscle-specific transcription of a gene operably linked to the synthetic promoter. Parts of the synthetic promoter may be naturally occurring (e.g. the minimal promoter or one or more CREs in the promoter), but the synthetic promoter as an entity is not naturally occurring.

As used herein, "minimal promoter" (also known as the "core promoter") refers to a typically short DNA segment which is inactive or largely inactive by itself, but can mediate transcription when combined with other transcription regulatory elements. Minimal promoter sequences can be derived from various different sources, including prokaryotic and eukaryotic genes. Examples of minimal promoters include the desmin minimum promoter, dopamine beta-hydroxylase gene minimum promoter, cytomegalovirus (CMV) immediate early gene minimum promoter (CMV-MP), and the herpes thymidine kinase minimal promoter (MinTK). A minimal promoter typically comprises the transcription start site (TSS) and elements directly upstream, a binding site for RNA polymerase II, and general transcription factor binding sites (often a TATA box). A minimal promoter may also include some elements downstream of the TSS, but these typically have little functionality absent additional regulatory elements.

As used herein, "proximal promoter" relates to the minimal promoter plus at least some additional regulatory sequence, typically the proximal sequence upstream of the gene that tends to contain primary regulatory elements. It often extends approximately 250 base pairs upstream of the TSS, and includes specific TFBS. A proximal promoter may also include one or more regulatory elements downstream of the TSS, for example a UTR or an intron. In the present case, the proximal promoter may suitably be a naturally occurring muscle-specific, cardiac muscle-specific, or skeletal muscle-specific proximal promoter that can be combined with one or more CREs or CRMs of the present invention. However, the proximal promoter can be synthetic.

As used herein, "promoter element" refers to either a minimal promoter or a proximal promoter as defined above. In the context of the present invention a promoter element is typically combined with one or more CREs and optionally with one or more additional regulatory elements in order to provide a synthetic muscle-specific, cardiac muscle-specific, or skeletal muscle-specific promoter of the present invention.

A "functional variant" of a CRE, CRM, promoter element, promoter or other regulatory nucleic acid in the context of the present invention is a variant of a reference sequence that retains the ability to function in the same way as the reference sequence, e.g. as a muscle-specific, cardiac muscle-specific, skeletal muscle-specific CRE, muscle-specific, cardiac muscle-specific, skeletal muscle-specific CRM or muscle-specific, cardiac muscle-specific, skeletal muscle-specific promoter. A "functional variant" of an additional regulatory element, de-targeting element, liver de-targeting element or intron is a variant of a reference sequence that retains the ability to function in the same way as the reference sequence e.g. as an additional regulatory element, de-targeting element, liver de-targeting element or intron. Alternative terms for such functional variants include "biological equivalents" or "equivalents".

It will be appreciated that the ability of a given CRE, CRM, promoter or other regulatory sequence to function as a muscle-specific, cardiac muscle-specific, or skeletal muscle-specific enhancer is determined significantly by the ability of the sequence to bind the same muscle-specific, cardiac muscle-specific, or skeletal muscle-specific TFs that bind to the reference sequence. Accordingly, in most cases, a functional variant of a CRE, CRM, promoter or other regulatory sequence will contain TFBS for the most or all of same TFs as the reference CRE, CRM, promoter or other regulatory sequence. It is preferred, but not essential, that the TFBS of a functional variant are in the same relative positions (i.e. order and general position) as the reference CRE, CRM, promoter or other regulatory sequence. It is also preferred, but not essential, that the TFBS of a functional variant are in the same orientation as the reference sequence (it will be noted that TFBS can in some cases be present in reverse orientation, e.g. as the reverse complement vis-à-vis the sequence in the reference sequence). It is also preferred, but not essential, that the TFBS of a functional variant are on the same strand as the reference sequence. Thus, in preferred embodiments, the functional variant comprises TFBS for the same TFs, in the same order, the same position, in the same orientation and on the same strand as the reference sequence. It will also be appreciated that the sequences lying between TFBS (referred to in some cases as spacer sequences, or suchlike) are of less consequence to the function of the CRE, CRM, promoter or other regulatory sequence. Such sequences can typically be varied considerably, and their lengths can be altered. However, in preferred embodiments the spacing (i.e. the distance between adjacent TFBS) is substantially the same (e.g. it does not vary by more than 20%, preferably by not more than 10%, and more preferably it is approximately the same) in a functional variant as it is in the reference sequence. It will be apparent that in some cases a functional variant of a CRE, CRM, promoter or other regulatory sequence can be present in the reverse orientation, e.g. it can be the reverse complement of a CRE, CRM, promoter or other regulatory sequence described above, or a variant thereof.

Levels of sequence identity between a functional variant and the reference sequence can also be an indicator or retained functionality. High levels of sequence identity in the TFBS of the CRE, CRM or promoter is of generally higher importance than sequence identity in the spacer sequences (where there is little or no requirement for any conservation of sequence). However, it will be appreciated that even within the TFBS, a considerable degree of sequence variation can be accommodated, given that the sequence of a functional TFBS does not need to exactly match the consensus sequence.

The ability of one or more TFs to bind to a TFBS in a given functional variant can be determined by any relevant means known in the art, including, but not limited to, electromobility shift assays (EMSA), binding assays, chromatin immunoprecipitation (ChIP), and ChIP-sequencing (ChIP-seq). In a preferred embodiment the ability of one or more TFs to bind a given functional variant is determined by EMSA. Methods of performing EMSA are well-known in the art. Suitable approaches are described in Sambrook et al. cited above. Many relevant articles describing this procedure are available, e.g. Hellman and Fried, Nat Protoc. 2007; 2(8): 1849-1861.

"Muscle-specific" or "muscle-specific expression" refers to the ability of a cis-regulatory element, cis-regulatory module or promoter to enhance or drive expression of a gene in muscle cells (or in muscle-derived cells) in a preferential or predominant manner as compared to other tissues (e.g. liver, kidney, spleen, heart, lung, and brain). Expression of the gene can be in the form of mRNA or protein. In preferred embodiments, muscle-specific expression is such that there is negligible expression in other (i.e. non-muscle) tissues or cells, i.e. expression is highly muscle-specific. For example, expression in muscle cells as opposed to other cells is at least 75%, 80%, 85%, 90% or 95%. "Cardiac muscle-specific" or "Cardiac muscle-specific expression" refers to the ability of a cis-regulatory element, cis-regulatory module, promoter element or promoter to enhance or drive expression of a gene in the cardiac muscle in a preferential or predominant manner as compared to other tissues (e.g. spleen, liver, lung, and brain) and compared to the skeletal muscle tissue. "Skeletal muscle-specific" or "Skeletal muscle-specific expression" refers to the ability of a cis-regulatory element, cis-regulatory module, promoter element or promoter to enhance or drive expression of a gene in the skeletal muscle in a preferential or predominant manner as compared to other tissues (e.g. spleen, liver, lung, and brain) and compared to the cardiac muscle tissue. There can be instances where lower degrees of specificity are desired and are part of this invention.

"CNS-specific" or "CNS-specific expression" refers to the ability of a promoter to enhance or drive expression of a gene in the central nervous system (CNS) cells (or in CNS-derived cells) in a preferential or predominant manner as compared to other tissues (e.g. liver, kidney, spleen, heart, lung, muscle and brain). Expression of the gene can be in the form of mRNA or protein. In preferred embodiments, CNS-specific expression is such that there is negligible expression in other (i.e. non-CNS) tissues or cells, i.e. expression is highly CNS-specific. For example, expression in CNS cells as opposed to other cells is at least 75%, 80%, 85%, 90% or 95%. There can be instances where lower degrees of specificity are desired and are part of this invention.

"Kidney-specific" or "Kidney-specific expression" refers to the ability of a promoter to enhance or drive expression of a gene in kidney cells (or in kidney-derived cells) in a preferential or predominant manner as compared to other tissues (e.g. liver, CNS, spleen, heart, lung, muscle and brain). Expression of the gene can be in the form of mRNA or protein. In preferred embodiments, kidney-specific expression is such that there is negligible expression in other (i.e. non-kidney) tissues or cells, i.e. expression is highly kidney-specific. For example, expression in kidney cells as opposed to other cells is at least 75%, 80%, 85%, 90% or 95%. There can be instances where lower degrees of specificity are desired and are part of this invention.

"Lung-specific" or "Lung-specific expression" refers to the ability of a promoter to enhance or drive expression of a gene in lung cells (or in lung-derived cells) in a preferential or predominant manner as compared to other tissues (e.g. liver, CNS, spleen, heart, muscle and brain). Expression of the gene can be in the form of mRNA or protein. In preferred embodiments, lung-specific expression is such that there is negligible expression in other (i.e. non-lung) tissues or cells, i.e. expression is highly lung-specific. For example, expression in lung cells as opposed to other cells is at least 75%, 80%, 85%, 90% or 95%. There can be instances where lower degrees of specificity are desired and are part of this invention.

The ability of a CRE, CRM or promoter to function as a muscle-specific, cardiac muscle-specific, or skeletal muscle-specific CRE, CRM or promoter can be readily assessed by the skilled person. The skilled person can thus easily determine whether any variant of the specific CRE, CRM or promoter recited above remains functional (i.e. it is a functional variant as defined above). For example, any given CRE or CRM to be assessed can be operably linked to a minimal promoter (e.g. positioned upstream of CMV-MP) and the ability of the CRE or CRM to drive muscle-specific, cardiac muscle-specific, or skeletal muscle-specific expression of a gene (typically a reporter gene) is measured. Alternatively, a variant of a CRE or CRM can be substituted into a synthetic muscle-specific, cardiac muscle-specific or skeletal muscle-specific promoter in place of a reference CRE or CRM, and the effects on muscle-specific, cardiac muscle-specific or skeletal muscle-specific expression driven by said modified promoter can be determined and compared to the unmodified form. Similarly, the ability of a promoter to drive muscle-specific, cardiac muscle-specific or skeletal muscle-specific expression can be readily assessed by the skilled person (e.g. as described in the examples below). Expression levels of a gene driven by a variant of a reference promoter can be compared to the expression levels driven by the reference promoter. In some embodiments, where muscle-specific, cardiac muscle-specific or skeletal muscle-specific expression levels driven by a variant promoter are at least 50%, at least 60%, at least 70%, at least 80%, at least 90%, or at least 100% of the expression levels driven by the reference promoter, it can be said that the variant remains functional. Suitable nucleic acid constructs and reporter assays to assess muscle-specific, cardiac muscle-specific or skeletal muscle-specific expression enhancement can easily be constructed, and the examples set out below give suitable methodologies.

Muscle-specificity, cardiac muscle-specificity or skeletal muscle-specificity can be identified wherein the expression of a gene (e.g. a therapeutic or reporter gene) occurs preferentially or predominantly in muscle-derived cells (or muscle tissue), cardiac muscle-derived cells (or cardiac tissue) or skeletal muscle-derived cells (or skeletal tissue). Preferential or predominant expression can be defined, for example, where the level of expression is greater, preferably significantly greater in muscle-derived, cardiac muscle-derived or skeletal muscle-derived cells than in other types of cells (i.e. non-muscle-derived cells, non-cardiac muscle-derived cells or non-skeletal muscle-derived cells). For example, expression in muscle-derived, cardiac muscle-derived or skeletal muscle-derived cells is suitably at least 5-fold higher than in non-muscle cells, non-cardiac muscle cells or non-skeletal muscle cells, preferably at least 10-fold higher than in non-muscle cells, non-cardiac muscle cells or non-skeletal muscle cells, and it may be 50-fold higher or more in some cases. For convenience, muscle-specific expression can suitably be demonstrated via a comparison of expression levels in a muscle cell line (e.g. muscle-derived cell line such as C2C12 or H2K cells (skeletal muscle) or H9C2 cells (cardiac), compared with expression levels in a liver-derived cell line (e.g. Huh7 or HepG2), kidney-derived cell line (e.g. HEK-293), a cervical tissue-derived cell line (e.g. HeLa) and/or a lung-derived cell line (e.g. A549). Cardiac muscle-specific expression can suitably be demonstrated via a comparison of expression levels in a cardiac muscle cell line (e.g. cardiac muscle derived cell line such as H9C2) or primary cardiomyocyte compared with expression levels in in a liver-derived cell line (e.g. Huh7 or HepG2), a kidney-derived cell line (e.g. HEK-293), a cervical tissue-derived cell line (e.g. HeLa), a lung-derived cell line (e.g. A549) and/or skeletal muscle-derived cells (e.g. C2C12 or H2K). Skeletal muscle-specific expression can suitably be demonstrated via a comparison of expression levels in a skeletal muscle-derived cells (e.g. C2C12 or H2K) or primary skeletal muscle cells compared with expression levels in in a liver-derived cell line (e.g. Huh7 or HepG2), a kidney-derived cell line (e.g. HEK-293), a cervical tissue-derived cell line (e.g. HeLa), a lung-derived cell line (e.g. A549) and/or cardiac muscle cell line (e.g. H9C2).

The synthetic muscle-specific, cardiac muscle-specific or skeletal muscle-specific promoters of the present invention preferably exhibit reduced expression in non-muscle-derived cells, suitably in Huh7, HEK-293, HeLa, and/or A549 cells when compared to a non-tissue specific promoter such as CMV-IE. The synthetic muscle-specific, cardiac muscle-specific or skeletal muscle-specific promoters of the present invention preferably have an activity of 50% or less than the CMV-IE promoter in non-muscle-derived cells (suitably in Huh7, HEK-293, HeLa, and/or A549 cells), suitably 25% or less, 20% or less, 15% or less, 10% or less, 5% or less or 1% or less. Generally, it is preferred that expression in non-muscle-derived cells is minimized, but in some cases this may not be necessary. Even if a synthetic promoter of the present invention has higher expression in, e.g., one or two non-muscle cells, as long as it generally has higher expression overall in a range of muscle cells versus non-muscle cell, it can still be a muscle-specific promoter. In some embodiments, a muscle-specific promoter expresses a gene at least 25%, or at least 35%, or at least 45%, or at least 55%, or at least 65%, or at least 75%, or at least 80%, or at least 85%, or at least 90%, or at least 95%, or any integer between 25%-95% higher in muscle cells as compared to non-muscle cells.

The synthetic muscle-specific promoters of the present invention are preferably suitable for promoting expression in the muscle of a subject, e.g. driving muscle-specific expression of a transgene, preferably a therapeutic transgene. The synthetic cardiac muscle-specific promoters of the present invention are preferably suitable for promoting expression in the heart of a subject, e.g. driving cardiac muscle-specific expression of a transgene, preferably a therapeutic transgene. The synthetic skeletal muscle-specific promoters of the present invention are preferably suitable for promoting expression in the skeletal muscles of a subject, e.g. driving skeletal muscle-specific expression of a transgene, preferably a therapeutic transgene. Preferred synthetic muscle-specific promoters of the present invention are suitable for promoting muscle-specific transgene expression and have an activity in muscle cells which is at least 15%, 20%, 30%, 40%, 50%, 60%, 70%, 80%, 90%, 100%, 125%, 150%, 175%, 200%, 250%, 300%, 350% or 400% of the activity of the CBA, CK7, CK8 promoter. In some embodiments, the synthetic muscle-specific promoters of the invention are suitable for promoting muscle-specific transgene expression at a level at least 100% of the activity of the CBA promoter, spc5-12 promoter, CK7 promoter, CK8 promoter preferably 150%, 200%, 300% or 500% of the activity of the CBA promoter, spc5-12 promoter, CK7 promoter, CK8 promoter. In some embodiments, the synthetic cardiac muscle-specific promoters of the invention are suitable for promoting cardiac muscle-specific transgene expression at a level at least 100% of the activity of the Tnnt2 or Myl2 promoter, preferably 150%, 200%, 300% or 500% of the activity of the Tnnt2 or Myl2 promoter. In some embodiments, the synthetic skeletal muscle-specific promoters of the invention are suitable for promoting skeletal muscle-specific transgene expression at a level at least 100% of the activity of the Tnnt2 or Myl2 promoter, preferably 150%, 200%, 300% or 500% of the activity of the spc5-12 promoter. Such muscle-specific expression is suitably determined in muscle-derived cells, e.g. as C2C12 or H2K cells (skeletal muscle) or H9C2 cells (cardiac) or primary muscle cells (suitably primary human myocytes). CK8 promoter is disclosed in Himeda, C.L., Chen, X., Hauschka, S.D. (2011). Design and Testing of Regulatory Cassettes for Optimal Activity in Skeletal and Cardiac Muscles. In: Duan, D. (eds) Muscle Gene Therapy. Methods in Molecular Biology, vol 709. Humana Press. https://doi.org/10.1007/978-1-61737-982-6_1

Synthetic muscle-specific, cardiac muscle-specific or skeletal muscle-specific promoters of the present invention may also be able to promote muscle-specific, cardiac muscle-specific or skeletal muscle-specific expression of a gene at a level at least 50%, 100%, 150% or 200% compared to CMV-IE in muscle-derived cells (e.g. C2C12 or H2K cells (skeletal muscle) or H9C2 cells (cardiac)).

The term "nucleic acid" as used herein typically refers to an oligomer or polymer (preferably a linear polymer) of any length composed essentially of nucleotides. A nucleotide unit commonly includes a heterocyclic base, a sugar group, and at least one, e.g. one, two, or three, phosphate groups, including modified or substituted phosphate groups. Heterocyclic bases may include, *inter alia,* purine and pyrimidine bases such as adenine (A), guanine (G), cytosine (C), thymine (T) and uracil (U) which are widespread in naturally-occurring nucleic acids, other naturally-occurring bases (e.g., xanthine, inosine, hypoxanthine) as well as chemically or biochemically modified (e.g., methylated), non-natural or derivatised bases. Sugar groups may include, *inter alia,* pentose (pentofuranose) groups such as preferably ribose and/or 2-deoxyribose common in naturally-occurring nucleic acids, or arabinose, 2-deoxyarabinose, threose or hexose sugar groups, as well as modified or substituted sugar groups. Nucleic acids as intended herein may include naturally occurring nucleotides, modified nucleotides or mixtures thereof. A modified nucleotide may include a modified heterocyclic base, a modified sugar moiety, a modified phosphate group or a combination thereof. Modifications of phosphate groups or sugars may be introduced to improve stability, resistance to enzymatic degradation, or some other useful property. The term "nucleic acid" further preferably encompasses DNA, RNA and DNA RNA hybrid molecules, specifically including hnRNA, pre-mRNA, mRNA, cDNA, genomic DNA, amplification products, oligonucleotides, and synthetic (e.g., chemically synthesised) DNA, RNA or DNA RNA hybrids. A nucleic acid can be naturally occurring, e.g., present in or isolated from nature; or can be non-naturally occurring, e.g., recombinant, i.e., produced by recombinant DNA technology, and/or partly or entirely, chemically or biochemically synthesised. A "nucleic acid" can be double-stranded, partly double stranded, or single-stranded. Where single-stranded, the nucleic acid can be the sense strand or the antisense strand. In addition, nucleic acid can be circular or linear.

The term "isolated", when referring to a nucleic acid, refers to a nucleic acid molecule devoid, in whole or part, of sequences normally associated with it in nature; or a sequence, as it exists in nature, but having heterologous sequences in association therewith; or a molecule disassociated from the chromosome.

The terms "identity" and "identical" and the like refer to the sequence similarity between two polymeric molecules, e.g., between two nucleic acid molecules, such as between two DNA molecules. Sequence alignments and determination of sequence identity can be done, e.g., using the Basic Local Alignment Search Tool (BLAST) originally described by Altschul et al. 1990 (J Mol Biol 215: 403-10), such as the "Blast 2 sequences" algorithm described by Tatusova and Madden 1999 (FEMS Microbiol Lett 174: 247-250).

Methods for aligning sequences for comparison are well-known in the art. Various programs and alignment algorithms are described in, for example: Smith and Waterman (1981) Adv. Appl. Math. 2:482; Needleman and Wunsch (1970) J. Mol. Biol. 48:443; Pearson and Lipman (1988) Proc. Natl. Acad. Sci. U.S.A. 85:2444; Higgins and Sharp (1988) Gene 73:237-44; Higgins and Sharp (1989) CABIOS 5:151-3; Corpet et al. (1988) Nucleic Acids Res. 16:10881-90; Huang et al. (1992) Comp. Appl. Biosci. 8:155-65; Pearson et al. (1994) Methods Mol. Biol. 24:307-31; Tatiana et al. (1999) FEMS Microbiol. Lett. 174:247-50. A detailed consideration of sequence alignment methods and homology calculations can be found in, e.g., Altschul et al. (1990) J. Mol. Biol. 215:403-10.

The National Center for Biotechnology information (NCBI) Basic Local Alignment Search Tool (BLAST^{™}; Altschul et al. (1990)) is available from several sources, including the National Center for Biotechnology Information (Bethesda, MD), and on the internet, for use in connection with several sequence analysis programs. A description of how to determine sequence identity using this program is available on the internet under the "help" section for BLAST^{™}. For comparisons of nucleic acid sequences, the "Blast 2 sequences" function of the BLAST^{™} (Blastn) program may be employed using the default parameters. Nucleic acid sequences with even greater similarity to the reference sequences will show increasing percentage identity when assessed by this method. Typically, the percentage sequence identity is calculated over the entire length of the sequence.

For example, a global optimal alignment is suitably found by the Needleman-Wunsch algorithm with the following scoring parameters: Match score: +2, Mismatch score: -3; Gap penalties: gap open 5, gap extension 2. The percentage identity of the resulting optimal global alignment is suitably calculated by the ratio of the number of aligned bases to the total length of the alignment, where the alignment length includes both matches and mismatches, multiplied by 100.

The term "hybridising" means annealing to two at least partially complementary nucleotide sequences in a hybridization process. In order to allow hybridisation to occur complementary nucleic acid molecules are generally thermally or chemically denatured to melt a double strand into two single strands and/or to remove hairpins or other secondary structures from single-stranded nucleic acids. The stringency of hybridisation is influenced by conditions such as temperature, salt concentration and hybridisation buffer composition. Conventional hybridisation conditions are described in, for example, Sambrook (2001) Molecular Cloning: a laboratory manual, 3rd Edition Cold Spring Harbor Laboratory Press, CSH, New York, but the skilled craftsman will appreciate that numerous different hybridisation conditions can be designed in function of the known or the expected homology and/or length of the nucleic acid sequence. High stringency conditions for hybridisation include high temperature and/or low sodium/salt concentration (salts include sodium as for example in NaCl and Na-citrate) and/or the inclusion of formamide in the hybridisation buffer and/or lowering the concentration of compounds such as SDS (sodium dodecyl sulphate detergent) in the hybridisation buffer and/or exclusion of compounds such as dextran sulphate or polyethylene glycol (promoting molecular crowding) from the hybridisation buffer. By way of non-limiting example, representative salt and temperature conditions for stringent hybridization are: 1 × SSC, 0.5% SDS at 65°C. The abbreviation SSC refers to a buffer used in nucleic acid hybridization solutions. One litre of a 20X (twenty times concentrate) stock SSC buffer solution (pH 7.0) contains 175.3 g sodium chloride and 88.2 g sodium citrate. A representative time period for achieving hybridisation is 12 hours.

The term "transcription factor binding site" (TFBS) is well known in the art. It will be apparent to the skilled person that alternative TFBS sequences can be used, provided that they are bound by the intended TF. Consensus sequences for the various TFBS are known in the art, and the skilled person can readily use this information to determine alternative TFBS. Furthermore, the ability of a TF to bind to a given putative sequence can readily be determined experimentally by the skilled person (e.g. by EMSA and other approaches well known in the art and discussed herein).

The meaning of "consensus sequence" is well-known in the art. In the present application, the following notation is used for the consensus sequences, unless the context dictates otherwise. Considering the following exemplary DNA sequence:
A[CT]N{A}YR

A means that an A is always found in that position; [CT] stands for either C or T in that position; N stands for any base in that position; and {A} means any base except A is found in that position. Y represents any pyrimidine, and R indicates any purine.

"Synthetic" in the present application means a nucleic acid molecule that does not occur in nature. Synthetic nucleic acids of the present invention are produced artificially, typically by recombinant technologies or *de novo* synthesis. Such synthetic nucleic acids may contain naturally occurring sequences (e.g. promoter, enhancer, intron, and other such regulatory sequences), but these are present in a non-naturally occurring context. For example, a synthetic gene (or portion of a gene) typically contains one or more nucleic acid sequences that are not contiguous in nature (chimeric sequences), and/or may encompass substitutions, insertions, and deletions and combinations thereof.

"Complementary" or "complementarity", as used herein, refers to the Watson-Crick base-pairing of two nucleic acid sequences. For example, for the sequence 5'-AGT-3' binds to the complementary sequence 3'-TCA-5'. Complementarity between two nucleic acid sequences may be "partial", in which only some of the bases bind to their complement, or it may be complete as when every base in the sequence binds to its complementary base. The degree of complementarity between nucleic acid strands has significant effects on the efficiency and strength of hybridisation between nucleic acid strands.

"Transfection" in the present application refers broadly to any process of deliberately introducing nucleic acids into cells, and covers introduction of viral and non-viral vectors, and includes or is equivalent to transformation, transduction and like terms and processes. Examples include, but are not limited to: transfection with viral vectors; transformation with plasmid vectors; electroporation (Fromm et al. (1986) Nature 319 :791-3) ; lipofection (Feigner et al. (1987) Proc. Natl. Acad. Sci. USA 84 :7413-7) ; microinjection (Mueller et al. (1978) Cell 15:579-85); Agrobacterium-mediated transfer (Fraley et al. (1983) Proc. Natl. Acad. Sci. USA 80:4803-7); direct DNA uptake; whiskers-mediated transformation; and microprojectile bombardment (Klein et al. (1987) Nature 327:70).

As used herein, the phrase "transgene" refers to an exogenous nucleic acid sequence. In one example, a transgene is a gene encoding an industrially or pharmaceutically useful compound, or a gene encoding a desirable trait. In yet another example, the transgene encodes useful nucleic acid such as an antisense nucleic acid sequence, wherein expression of the antisense nucleic acid sequence inhibits expression of a target nucleic acid sequence. The transgene preferably encodes a therapeutic product, e.g. a protein.

The term "vector" is well known in the art, and as used herein refers to a nucleic acid molecule, e.g. double-stranded DNA, which may have inserted into it a nucleic acid sequence according to the present invention. A vector is suitably used to transport an inserted nucleic acid molecule into a suitable host cell. A vector typically contains all of the necessary elements that permit transcribing the inserted nucleic acid molecule, and, preferably, translating the transcript into a polypeptide. A vector typically contains all of the necessary elements such that, once the vector is in a host cell, the vector can replicate independently of, or coincidental with, the host chromosomal DNA; several copies of the vector and its inserted nucleic acid molecule may be generated. Vectors of the present invention can be episomal vectors (i.e., that do not integrate into the genome of a host cell), or can be vectors that integrate into the host cell genome. This definition includes both non-viral and viral vectors. Non-viral vectors include but are not limited to plasmid vectors (e.g. pMA-RQ, pUC vectors, bluescript vectors (pBS) and pBR322 or derivatives thereof that are devoid of bacterial sequences (minicircles)) transposons-based vectors (e.g. PiggyBac (PB) vectors or Sleeping Beauty (SB) vectors), etc. Larger vectors such as artificial chromosomes (bacteria (BAC), yeast (YAC), or human (HAC)) may be used to accommodate larger inserts. Viral vectors are derived from viruses and include but are not limited to retroviral, lentiviral, adeno-associated viral, adenoviral, herpes viral, hepatitis viral vectors or the like. Typically, but not necessarily, viral vectors are replication-deficient as they have lost the ability to propagate in a given cell since viral genes essential for replication have been eliminated from the viral vector. However, some viral vectors can also be adapted to replicate specifically in a given cell, such as e.g. a cancer cell, and are typically used to trigger the (cancer) cell-specific (onco)lysis. Virosomes are a non-limiting example of a vector that comprises both viral and non-viral elements, in particular they combine liposomes with an inactivated HIV or influenza virus (Yamada et al., 2003). Another example encompasses viral vectors mixed with cationic lipids.

The term "operably linked", "operably connected" or equivalent expressions as used herein refer to the arrangement of various nucleic acid elements relative to each other such that the elements are functionally connected and are able to interact with each other in the manner intended. Such elements may include, without limitation, a promoter, a CRE (e.g. enhancer or other regulatory element), a de-targeting element (e.g., liver de-targeting element), a promoter element, a polyadenylation sequence, one or more introns and/or exons, and a coding sequence of a gene of interest to be expressed. The nucleic acid sequence elements, when properly oriented or operably linked, act together to modulate the activity of one another, and ultimately may affect the level of expression of an expression product. By modulate is meant increasing, decreasing, or maintaining the level of activity of a particular element. The position of each element relative to other elements may be expressed in terms of the 5' terminus and the 3' terminus of each element or their position upstream or downstream of another element or position (such as a TSS or promoter element), and the distance between any particular elements may be referenced by the number of intervening nucleotides, or base pairs, between the elements. As understood by the skilled person, operably linked implies functional activity, and is not necessarily related to a natural positional link. Indeed, when used in nucleic acid expression cassettes, CREs will typically be located immediately upstream of the promoter element (although this is generally the case, it should definitely not be interpreted as a limitation or exclusion of positions within the nucleic acid expression cassette), but this needs not be the case *in vivo,* e.g., a regulatory element sequence naturally occurring downstream of a gene whose transcription it affects is able to function in the same way when located upstream of the promoter. Hence, according to a specific embodiment, the regulatory or enhancing effect of the regulatory element can be position- independent.

A "spacer sequence" or "spacer" as used herein is a nucleic acid sequence that separates two functional nucleic acid sequences (e.g. TFBS, CREs, CRMs, promoter element, etc.). It can have essentially any sequence, provided it does not prevent the functional nucleic acid sequence (e.g. cis-regulatory element) from functioning as desired (e.g. this could happen if it includes a silencer sequence, prevents binding of the desired transcription factor, or suchlike). Typically, it is non-functional, as in it is present only to space adjacent functional nucleic acid sequences from one another. In some embodiments, spacers may have a length of 75, 50, 40, 30, 30 or 10 nucleotides or fewer.

The term "pharmaceutically acceptable" as used herein is consistent with the art and means compatible with the other ingredients of the pharmaceutical composition and not deleterious to the recipient thereof.

"Therapeutically effective amount" and like phrases mean a dose or plasma concentration in a subject that provides the desired specific pharmacological effect, e.g. to express a therapeutic gene in the muscle. A therapeutically effective amount may not always be effective in treating the conditions described herein, even though such dosage is deemed to be a therapeutically effective amount by those of skill in the art. The therapeutically effective amount may vary based on the route of administration and dosage form, the age and weight of the subject, and/or the disease or condition being treated.

The term "AAV vector" as used herein is well known in the art, and generally refers to an AAV vector nucleic acid sequence including various nucleic acid sequences. An AAV vector as used herein typically comprise a heterologous nucleic acid sequence not of AAV origin as part of the vector. This heterologous nucleic acid sequence typically comprises a promoter as disclosed herein as well as other sequences of interest for the genetic transformation of a cell. In general, the heterologous nucleic acid sequence is flanked by at least one, and generally by two AAV inverted terminal repeat sequences (ITRs).

An "AAV virion" or "AAV virus" or "AAV viral particle" or "AAV vector particle" refers to a viral particle composed of at least one AAV capsid polypeptide (including both variant AAV capsid polypeptides and non-variant parent capsid polypeptides) and an encapsidated polynucleotide AAV vector. If the particle comprises a heterologous nucleic acid (i.e. a polynucleotide other than a wild-type AAV genome, such as a transgene to be delivered to a mammalian cell), it can be referred to as an "AAV vector particle" or simply an "AAV vector". Thus, production of AAV virion or AAV particle necessarily includes production of AAV vector as such a vector is contained within an AAV virion or AAV particle.

A "small interfering" or "short interfering RNA" or siRNA is a RNA duplex of nucleotides targeted to a gene interest (a "target gene"). An "RNA duplex" refers to the structure formed by the complementary pairing between two regions of a RNA molecule. siRNA is "targeted" to a gene and the nucleotide sequence of the duplex portion of the siRNA is complementary to a nucleotide sequence of the targeted gene. In some embodiments, the length of the duplex of siRNAs is less than 30 nucleotides. In some embodiments, the duplex can be 29, 28, 27, 26, 25, 24, 23, 22, 21, 20, 19, 18, 17, 16, 15, 14, 13, 12, 11 or 10 nucleotides in length. In some embodiments, the length of the duplex is 19- 25 nucleotides in length. The RNA duplex portion of the siRNA can be part of a hairpin structure. In addition to the duplex portion, the hairpin structure may contain a loop portion positioned between the two sequences forming the duplex. The loop can vary in length. In some embodiments the loop is 5, 6, 7, 8, 9, 10, 11, 12 or 13 nucleotides in length. The hairpin structure can also contain 3' or 5' overhang portions. In some embodiments, the overhang is a 3' or a 5' overhang 0, 1, 2, 3, 4 or 5 nucleotides in length.

The terms "treatment" or "treating" refer to reducing, ameliorating or eliminating one or more signs, symptoms, or effects of a disease or condition. "Treatment," as used herein thus includes any treatment of a disease in a mammal, particularly in a human, and includes: (a) preventing the disease from occurring in a subject predisposed to the disease or at risk of acquiring the disease but has not yet been diagnosed as having it; (b) inhibiting the disease, i.e., arresting its development; and (c) relieving the disease, i.e., causing regression of the disease.

The "administration" of an agent to a subject includes any route of introducing or delivering to a subject the agent to perform its intended function. Administration can be carried out by any suitable route, including orally, intranasally, intraocularly, ophthalmically, parenterally (intravenously, intramuscularly, intraperitoneally, or subcutaneously), or topically. Administration includes self-administration and the administration by another. Intramuscular administration is of particular interest in the present invention.

The terms "individual," "subject," and "patient" are used interchangeably, and refer to any individual subject with a disease or condition in need of treatment. For the purposes of the present disclosure, the subject may be a primate, preferably a human, or another mammal, such as a dog, cat, horse, pig, goat, or bovine, and the like.

The term "gene therapy vector" as used herein refers to a vector, as defined above, which is suitable for or intended for gene therapy. Gene therapy vectors typically comprise a promoter operably linked to a therapeutically useful expression product (e.g. a transgene which is useful in treating a disease or condition).

The term "de-targeting element" as used herein refers to a nucleic acid sequence which when added to a CRM, synthetic promoter, expression cassette or a vector functions to reduce expression of the CRM, synthetic promoter, expression cassette or a vector in a specific tissue or cell. Examples of de-targeting elements may be found in Kopp F, Schnoedt M, Haase R, Wagner E, Roidl A, Ogris M. De-targeting by miR-143 decreases unwanted transgene expression in non-tumorigenic cells. Gene Ther. 2013 Nov;20(11):1104-9. doi: 10.1038/gt.2013.37. Epub 2013 Jun 27. PMID: 23804075, Dhungel B, Ramlogan-Steel CA, Layton CJ, Steel JC. MicroRNA199a-Based Post-transcriptional Detargeting of Gene Vectors for Hepatocellular Carcinoma. Mol Ther Nucleic Acids. 2018 Dec 7;13:78-88. doi: 10.1016/j.omtn.2018.08.016. Epub 2018 Aug 24. PMID: 30245470; PMCID: PMC6148835 (particularly figure 2 and the materials and methods) and Dhungel, B., Ramlogan-Steel, C.A. & Steel, J.C. Synergistic and independent action of endogenous microRNAs 122a and 199a for post-transcriptional liver detargeting of gene vectors. Sci Rep 8, 15539 (2018). https://doi.org/10.1038/s41598-018-33801-4 (particularly figure 2), Juliette Hordeaux, Elizabeth L. Buza, Brianne Jeffrey, Chunjuan Song, Tahsin Jahan and Yuan Yuan, Yanqing Zhu, Peter Bell, Mingyao Li, Jessica A. Chichester, Roberto Calcedo and James M. Wilson, MicroRNA-mediated inhibition of transgene expression reduces dorsal root ganglion toxicity by AAV vectors in primates , Science Translational Medicine, Volume 12 , 569, 2020, doi 10.1126/scitranslmed.aba9188, which are incorporated herein by reference. The de-targeting element may be a binding site for a protein or miRNA which is highly expressed in a specific cell or tissue. In some embodiments, the de-targeting element may be a liver de-targeting element. Addition of a liver de-targeting element to a CRM, synthetic promoter, expression cassette or a vector may reduce the expression of the CRM, synthetic promoter, expression cassette or a vector in liver tissue or cell.

The invention will now be described with reference to the following non-limiting examples:

### Example 1 - In vivo data

The strength of the synthetic muscle-specific promoters according to embodiments of this invention are tested by operably linking each synthetic muscle-specific promoter to the reporter gene luciferase.

### Materials and methods

A selection of the synthetic muscle-specific promoters are tested *in vivo.*

### In vivo experiment

AAVs comprising a synthetic promoter (e.g. SP0524) operably linked to luciferase are diluted in 0.9% saline and delivered via tail vein injection in 8-week-old male Balb/c mice at 1e¹¹ vg/ 200µl per mouse (6 mice per group). Mice are sacrificed 6 weeks post injection by schedule 1 method. AAV9 is used.

Diaphragm (skeletal muscle), heart (cardiac muscle), gastrocnemius (skeletal muscle), soleus (skeletal muscle), tibialis anterior (TA) (skeletal muscle) and liver are collected from each mouse at 6 weeks post injection. The following tissues are collected for IHC: heart, TA, soleus, liver, gastrocnemius. For luciferase (Luc) expression and vector copy number (VCN) analysis, all samples are snap-frozen in liquid nitrogen immediately after dissection, then moved to dry ice before being stored at -80°C. Tissues for IHC are slowly frozen in alcohol containing liquid nitrogen and then moved to dry ice. Gastrocnemius muscle comprises fast and slow twitch fibres at roughly 1:1 ratio.

### Live imaging

All 6 mice from each group are imaged at week 4 post-injection to check the expression of luciferase. For imaging, the IVIS^{®} Spectrum Lumina III series in vivo imaging system is used. IVIS machine detects bioluminescence by imaging the light emitted by enzyme-catalysed reactions to report activity at the molecular level. Bioluminescent reporters require a small chemical substrate for non-invasive imaging in cell biology and small animal studies. This protocol specifically requires IP injection of D-Luciferin and anaesthesia induction by isofane. The procedure for imaging is as follows:
- A stock solution of D-Luciferin potassium salt (as a substrate) is prepared by using Luciferin from (cat# MB000102, Syd Labs, USA) Lot# Ro405-017, and (cat#LUCK-1G, GoldBio, USA) Lot#016067LUCK.For reconstitution, PBS from Gibco Cat#14190-094, Lot#2241142 is used.
- Luciferin stock is prepared 15 mg/ml (W/V), (1500 mg/ 100 ml of PBS), aliquoted, labelled and wrapped up with foil to protect from light and kept in -20/-80°C freezer.
- D-luciferin substrate is administered intraperitoneally into mice at 300 µl /mouse of Luciferin stock.
- The anesthetic mixer is topped up with Isoflurane / Isoflurane.
- The mice are moved into the anaesthetic induction chamber.
- The oxygen supply is set at 2.5L /minute and mice are observed while they were going to sleep.
- Once the mice are under the anesthetic and during 5 minute countdown the mice are moved from the induction chamber to bioimager IVIS chamber in order corresponding to animal experimental number (Group).
- Images are taken.
- Luciferase expression is compared between the top half of the animals (comprising heart, diaphragm and liver) and the bottom half of the animals (comprising soleus, tibialis anterior and gastrocnemius)

### Tissue homogenization and lysis

Tissues stored at -80°C, are thawed at room temperature. Reporter lysis buffer (Promega, Catalog number #E4030) is pipetted into each tube containing the sample tissue. 1-2 Qiagen Carbide Beads (QIAGEN, Catalog number #69997) are added per tube. The tubes are placed in a Qiagen Tissue Lyser II (QIAGEN, Catalog number#85220) and homogenized at a frequency of 25.0 for 75 sec. After 75 sec, the adaptor is rotated and the samples are homogenised for a further 75 sec. The samples are then snap frozen at -80°C for 10-15 mins, followed by thawing at 37°C for 10 mins using a dry heating bath system. The tubes are centrifuged at 10,000xg for 3 mins. The supernatant is transferred to a fresh 1.5ml Eppendorf tube, without disturbing the pellet. The above steps are repeated a second time by adding extra reporter lysis buffer to the pelleted tissues. The resulting supernatant from the repeated process is added to the same 1.5ml Eppendorf tube. The tubes are vortexed well and stored at -80°C.

### Measurement of luciferase activity

- Luciferase activity is measured using Promega's Luciferase assay system (Promega, Catalog number #E4550)
- The working luciferase (LAR) reagent solution is thawed at room temperature, 30 minutes before use, while the sample tissue lysates are thawed on ice prior to use
- Using the GloMax^{®} Navigator Microplate Luminometer, (Promega, Catalog number #GM2000) a linear range of light detection is determined specifically by setting up a standard curve using QuantiLum Recombinant Luciferase, (1mg/ml, Promega, Catalog number #E1701). The luciferase stock solution is serially diluted 10-fold using reporter lysis buffer in 1.5mL tubes from 10⁻¹mg/ml to 10⁻⁹mg/ml
- Samples of interest are diluted 1:100 in fresh 1.5ml Eppendorf tubes
- 10µl lysates of each standard and diluted sample of interest are manually pipetted into 96 well flat bottom solid white plate - in triplicate, and luminescence (RLU) is measured by injection of 50µl of LAR into each well on the GlowMax reader

### Protein extraction and quantification

Protein is extracted from the collected tissues and quantified using a BCA Pierce protein assay kit ((ThermoFisher 23225). The manufacturer's instructions are as follows. A bovine serum albumin (BSA) standard is prepared by diluting albumin to a working range of 20-20000 µg/ml. The appropriate volume of the BCA working reagent (WR) is prepared by mixing 50 parts of BCA Reagent A with 1 part of BCA Reagent B (50:1, Reagent A:B). The microplate procedure (sample to WR ratio of 1:8) is followed:
- 25 µL of each standard or unknown sample replicate is pipetted into a well of a 96 well plate
- 200 µL of the WR is added to each well and the plate is placed on a plate shaker for 30 seconds to allow mixing
- The plate is covered and incubated for 30 minutes at 37°C
- The plate is cooled to room temperature and absorbance is measured at 562 nm on a BMG fluostar plate reader

The average 562 nm absorbance measurement of the blank standard replicates is subtracted from the 562 nm absorbance measurements of all other BSA standard replicates and sample replicates. A standard curve is prepared by plotting the average blank-corrected 562 nm measurement for each BSA standard against its concentration in µg/ml. The standard curve is used to determine the protein concentration of each sample.

### Vector copy number

Vector copy number is determined by dual Taqman qPCR. DNA is extracted using the DNeasy^{®} Blood & Tissue Kit (250), (QIAGEN, Catalog number #69506). Taqman qPCR is performed on each sample using both Luciferase and GAPDH -specific primer and probe sets:

| **Target** | **Primers** | **Probe** |
|---|---|---|
| Luc2 | Fw (5' ACGCTGGGCTACTTGATC 3') (SEQ ID NO: 23) | 5' TTTCGGGTCGTGCTCATG 3' (SEQ ID NO: 24) |
| | RV (5' CAAGAATAGCTCCTCCTCG 3') (SEQ ID NO: 25) | FAM/BHQ1 (5' reporter dye/3' quencher) |
| mGapdH | FW (5' ACGGCAAATTCAACGGCAC 3') (SEQ ID NO: 5) | 5' TTGTCATCAACGGGAAGCCCATCA 3' (SEQ ID NO: 6) |
| | RV (5' TAGTGGGGTCTCGCTCCTGG 3') (SEQ ID NO: 18) | JOE/BHQ1 (5' reporter dye/3' quencher) |

Standard curves are used for Luc and GAPDH for analysis purposes. In the multiplex qPCR protocol, the following final concentrations of reagents and DNA are used: Luc2 fw Primer (350nM), Luc2 RV Primer (350nM), mGapdH FW Primer (350nM), mGapdH RV Primer (350nM), Luc2 Probe (250nM), mGapdH Probe (250nM) and DNA (10ng/uL). The PCR cycle protocol is as follows: 95C - 20 seconds, PCR: 40 cycles, 95C - 1 second, 60C - 20 seconds. The ΔΔCt VCN (Quantity) per genome is calculated by subtracting the average VCN (Quantity) per genome for the saline samples (threshold).

### Results

SP0524 drives high expression in cardiac muscle (heart). SP0524 drives high expression in skeletal muscle (e.g. tibialis anterior, diaphragm). SP0524 drives lower expression in liver.

SP0525 drives high expression in cardiac muscle (heart) and drives high expression in skeletal muscle (e.g. gastrocnemius, soleus, tibialis anterior). SP0525 drives lower expression in liver.

SP0526 drives high expression in cardiac muscle (heart) and drives high expression in skeletal muscle (e.g. gastrocnemius, tibialis anterior, diaphragm). SP0526 drives lower expression in liver.

SP0527 drives high expression in cardiac muscle (heart) and drives high expression in skeletal muscle (e.g. gastrocnemius, tibialis anterior, diaphragm). SP0527 drives lower expression in liver.

SP0528 drives high expression in cardiac muscle (heart) and drives high expression in skeletal muscle (e.g. gastrocnemius, tibialis anterior, diaphragm). SP0528 drives lower expression in liver.

### Sequence Information

**Table 1 - Muscle-specific synthetic promoters**

| | | |
|---|---|---|
| SP0525 (SEQ ID NO: 1) | gctgcccatgtaaggaggcaaggcctggggacacccgagatgcctggttataattaacc cagacatgtggctgccccccccccccaacacctgctgcctctaaaaataaccctgttctcct ctataaatacccgctctggtatttggggttggcagctgttgaggatacaagtgacccctgctc tgttaattattggcaaattgcctaacttcaacgtaaggaaatagagtcatatgtttgctcactg aaggttactagttgtacttatataagggggtgggggcgcgttcgtcctcagtcgcgatcgaa cactcgagccgagcagacgtgcctacggaccggccacc | 348 |
| SP0526 (SEQ ID NO: 2) | gctgcccatgtaaggaggcaaggcctggggacacccgagatgcctggttataattaacc cagacatgtggctgccccccccccccaacacctgctgcctctaaaaataaccctgttctcct ctataaatacccgctctggtatttggggttggcagctgttgttggcaaattgcctaacttcaac gtaaggaaatagagtcatatgtttgctcacgtacttatataagggggtgggggcgcgttcgt cctcagtcgcgatcgaacactcgagccgagcagacgtgcctacggaccggccacc | 302 |
| SP0527 (SEQ ID NO: 3) | gctgcccatgtaaggaggcaaggcctggggacacccgagatgcctggttataattaacc cagacatgtggctgccccccccccccaacacctgctgcctctaaaaataaccctgttctcct ctataaatacccgctctggtatttggggttggcagctgttggcctaacttcaacgtaaggaaa tagagtcatgtacttatataagggggtgggggcgcgttcgtcctcagtcgcgatcgaacact cgagccgagcagacgtgcctacggaccggccacc | 280 |
| SP0528 SEQ ID NO: 4) | gctgcccatgtaaggaggcaaggcctggggacacccgagatgcctggttataattaacc cagacatgtggctgccccccccccccaacacctgctgcctctaaaaataaccctggccta | 311 |
| | acttcaacgtaaggaaatagagtcatttctcctctataaatacccgctctggtatttggggttg gcagctgttggcctaacttcaacgtaaggaaatagagtcatgtacttatataagggggtgg gggcgcgttcgtcctcagtcgcgatcgaacactcgagccgagcagacgtgcctacggac cggccacc | |
| SP0524 (SEQ ID NO: 7) | gctgcccatgtaaggaggcaaggcctggggacacccgagatgcctggttataattaacc cagacatgtggctgccccccccccccaacacctgctgcctctaaaaataaccctgttctcct ctataaatacccgctctggtatttggggttggcagctgttggtacttatataagggggtgggg gcgcgttcgtcctcagtcgcgatcgaacactcgagccgagcagacgtgcctacggaccg gccacc | 249 |

**Table 2 - CRMs from synthetic promoters of Table 1**

| | | |
|---|---|---|
| CRM from SP0524, SP0530 and SP0531 (SEQ ID NO: 8) | gctgcccatgtaaggaggcaaggcctggggacacccgagatgcctggttataat taacccagacatgtggctgccccccccccccaacacctgctgcctctaaaaata accctgttctcctctataaatacccgctctggtatttggggttggcagctgttg | 162 |
| CRM_SP0525 (SEQ ID NO: 20) | gctgcccatgtaaggaggcaaggcctggggacacccgagatgcctggttataat taacccagacatgtggctgccccccccccccaacacctgctgcctctaaaaata accctgttctcctctataaatacccgctctggtatttggggttggcagctgttgaggat acaagtgacccctgctctgttaattattggcaaattgcctaacttcaacgtaaggaa atagagtcatatgtttgctcactgaaggttactagtt | 261 |
| CRM_SP0526 (SEQ ID NO: 21) | gctgcccatgtaaggaggcaaggcctggggacacccgagatgcctggttataat taacccagacatgtggctgccccccccccccaacacctgctgcctctaaaaata accctgttctcctctataaatacccgctctggtatttggggttggcagctgttgttggca aattgcctaacttcaacgtaaggaaatagagtcatatgtttgctcac | 215 |
| CRM_SP0527 (SEQ ID NO: 22) | gctgcccatgtaaggaggcaaggcctggggacacccgagatgcctggttataat taacccagacatgtggctgccccccccccccaacacctgctgcctctaaaaata accctgttctcctctataaatacccgctctggtatttggggttggcagctgttggccta acttcaacgtaaggaaatagagtcat | 193 |
| CRM_SP0528 (SEQ ID NO: 9) | gctgcccatgtaaggaggcaaggcctggggacacccgagatgcctggttataat taacccagacatgtggctgccccccccccccaacacctgctgcctctaaaaata accctggcctaacttcaacgtaaggaaatagagtcatttctcctctataaatacccg ctctggtatttggggttggcagctgttggcctaacttcaacgtaaggaaatagagtc at | 224 |

**Table 3 - CREs from synthetic promoters of Table 1**

| | | |
|---|---|---|
| CRE0145 (SEQ ID NO: 10) | gctgcccatgtaaggaggcaaggcctggggacacccgagatgcctggttataattaa cccagacatgtggctgccccccccccccaacacctgctgcctctaaaaataaccctg | 114 |
| DES_MT_enhan cer_48bp (SEQ ID NO: 11) | ttctcctctataaatacccgctctggtatttggggttggcagctgttg | 48 |

**Table 4 - Promoter elements**

| | | |
|---|---|---|
| SCP1 (SEQ ID NO: 12) | gtacttatataagggggtgggggcgcgttcgtcctcagtcgcgatcgaacactcgagc cgagcagacgtgcctacggaccg | 81 |
| CRE0053 (SEQ ID NO: 26) | caccgcctgctgccacggccggccgtataaatagaggcgaggagcagctgggctct cttggcagtcacc | 69 |

**Table 5 - Liver de-targeting elements from synthetic promoters of Table 1**

| | | |
|---|---|---|
| Liver de-targeting sequence 1 (SEQ ID NO: 13) | aggatacaagtgacccctgctctgttaattattggcaaattgcctaacttcaa cgtaaggaaatagagtcatatgtttgctcactgaaggttactagtt | 99 |
| Liver de-targeting sequence 2 (SEQ ID NO: 14) | ttggcaaattgcctaacttcaacgtaaggaaatagagtcatatgtttgctcac | 53 |
| Liver de-targeting sequence 3 (SEQ ID NO: 17) | gcctaacttcaacgtaaggaaatagagtcat | 31 |
| ZBTB20 binding site (SEQ ID NO: 15) | cttcaacgtaaggaaatag | 19 |
| Mir122 miRNA target sequence 1 (SEQ ID NO: 16) | caaacaccattgtcacactccaTCACcaaacaccattgtcacactccaG ATATCcaaacaccattgtcacactcca | 76 |
| Mir122 miRNA target sequence 2 (SEQ ID NO: 19) | caaacaccattgtcacactcca | 22 |

**Table 6 - Schematic representation of the muscle-specific promoters according to embodiments of this invention with the cis-regulatory elements, promoter elements and other elements indicated**

| | | | | | |
|---|---|---|---|---|---|
| SP0525 | CRE0145 | DES_MT_enhancer _48bp | Liver de-targeting sequence 1 | SCP1 | |
| SP0526 | CRE0145 | DES_MT_enhancer _48bp | Liver de-targeting sequence 2 | SCP1 | |
| SP0527 | CRE0145 | DES_MT_enhancer _48bp | Liver de-targeting sequence 3 | SCP1 | |
| SP0528 | CRE0145 | Liver de-targeting sequence 3 | DES_MT_enhance r_48bp | Liver de-targeting sequence 3 | SCP1 |

**Table 7 - Schematic representation of the muscle-specific expression cassettes with liver de-targeting element**

| | | | | | |
|---|---|---|---|---|---|
| Muscle-specific promoter | Expression product (e.g. gene of interest) | Mir122 miRNA target sequence 1 | polyA signal | | |
| Muscle-specific promoter | Expression product (e.g. gene of interest) | T2A | Expression product (e.g. Second gene of interest) | Mir122 miRNA target sequence 1 | polyA signal |
| SP0524 | Expression product (e.g. gene of interest) | Mir122 miRNA target sequence 1 | polyA signal | | |
| SP0524 | Expression product (e.g. gene of interest) | T2A | Expression product (e.g. Second gene of interest) | Mir122 miRNA target sequence 1 | polyA signal |

### Clauses

1. A synthetic muscle-specific cis-regulatory module (CRM) comprising CRE0145 or a functional variant thereof, DES_MT enhancer 48bp or a functional variant thereof and at least one de-targeting element or functional variant thereof.
2. The synthetic muscle-specific CRM according to clause 1 wherein the at least one de-targeting element is a liver de-targeting element or a functional variant thereof.
3. The synthetic muscle-specific CRM according to clause 2 wherein the liver de-targeting element or a functional variant thereof is selected from the group consisting of:
   - Liver de-targeting sequence 1 (SEQ ID NO: 13) or a functional variant thereof;
   - Liver de-targeting sequence 2 (SEQ ID NO: 14) or a functional variant thereof;
   - Liver de-targeting sequence 3 (SEQ ID NO: 17) or a functional variant thereof;
   - ZBTB20 binding site (SEQ ID NO: 15) or a functional variant thereof;
   - Mir122 miRNA target sequence 1 (SEQ ID NO: 16) or a functional variant thereof; and
   - Mir122 miRNA target sequence 2 (SEQ ID NO: 19) or a functional variant thereof.
4. The synthetic muscle-specific CRM according to any preceding clause wherein the CRM comprises a combination of regulatory elements, or functional variants thereof, selected from the group consisting of:
   - CRE0145, DES_MT_enhancer_48bp and Liver de-targeting sequence 1;
   - CRE0145, DES_MT_enhancer_48bp and Liver de-targeting sequence 2;
   - CRE0145, DES_MT_enhancer_48bp and Liver de-targeting sequence 3;
   - CRE0145, DES_MT enhancer 48bp and ZBTB20 binding site;
   - CRE0145, Liver de-targeting sequence 3, DES_MT enhancer 48bp and Liver de-targeting sequence 3; and
   - CRE0145, ZBTB20 binding site, DES_MT enhancer 48bp and ZBTB20 binding site, optionally wherein the regulatory elements are present in the CRM in the recited order and adjacent to each other.
5. The synthetic muscle-specific CRM according to any preceding clause comprising or consisting of SEQ ID NO: 20, 21, 22 and 9 or a functional variant thereof, optionally comprising or consisting of a sequence which is at least 70%, 75%, 80%, 85%, 90%, 95%, 96%, 97%, 98% or 99% identical to SEQ ID NO: 20, 21, 22 and 9.
6. A synthetic muscle-specific promoter comprising a CRM according to any preceding clause operably linked to a promoter element, optionally wherein the promoter element is SCP1 or a functional variant thereof or CRE0053 or a functional variant thereof.
7.The synthetic muscle-specific promoter according to clause 6 comprising a synthetic muscle-specific CRM comprising CRE0145 or a functional variant thereof, DES_MT enhancer 48bp or a functional variant thereof and at least one a liver de-targeting element selected from the group consisting of:
   - Liver de-targeting sequence 1 (SEQ ID NO: 13) or a functional variant thereof;
   - Liver de-targeting sequence 2 (SEQ ID NO: 14) or a functional variant thereof;
   - Liver de-targeting sequence 3 (SEQ ID NO: 17) or a functional variant thereof; and
   - ZBTB20 binding site (SEQ ID NO: 15) or a functional variant thereof;
   operably linked to a promoter element SCP1 (SEQ ID NO: 12) or a functional variant thereof or promoter element CRE0053 (SEQ ID NO: 26) or a functional variant thereof.
8. The synthetic muscle-specific promoter according to any one of clauses 6-7 comprising synthetic muscle-specific CRM comprising a combination of regulatory elements, or functional variants thereof, selected from the group consisting of:
   - CRE0145, DES_MT_enhancer_48bp and Liver de-targeting sequence 1;
   - CRE0145, DES_MT_enhancer_48bp and Liver de-targeting sequence 2;
   - CRE0145, DES_MT_enhancer_48bp and Liver de-targeting sequence 3;
   - CRE0145, DES_MT_enhancer_48bp and ZBTB20 binding site;
   - CRE0145, Liver de-targeting sequence 3, DES_MT enhancer 48bp and Liver de-targeting sequence 3; and
   - CRE0145, ZBTB20 binding site, DES_MT_enhancer_48bp and ZBTB20 binding site;
   operably linked to a promoter element SCP1 (SEQ ID NO: 12) or a functional variant thereof or promoter element CRE0053 (SEQ ID NO: 26) or a functional variant thereof,
   optionally wherein the regulatory elements are present in the CRM in the recited order and adjacent to each other.
9. The synthetic muscle-specific promoter according to any one of clauses 6-8 wherein the synthetic muscle-specific promoter comprises or consists of SP0525 (SEQ ID NO: 1), SP0526 (SEQ ID NO: 2), SP0527 (SEQ ID NO: 3) or SP0528 (SEQ ID NO: 4) or a functional variant of any thereof.
10. The synthetic muscle-specific promoter according to any one of clauses 6-9 comprising or consisting of a sequence which is at least 70%, 75%, 80%, 85%, 90%, 95%, 96%, 97%, 98% or 99% identical to any one of SEQ ID NO: 1-4.
11. The synthetic muscle-specific promoter according to any one of clauses 6-10 wherein the synthetic muscle-specific promoter comprises or consists of SP0527 (SEQ ID NO: 3) or a functional variant of any thereof, optionally wherein the synthetic muscle-specific promoter comprising or consisting of a sequence which is at least 70%, 75%, 80%, 85%, 90%, 95%, 96%, 97%, 98% or 99% identical to SEQ ID NO: 3.
12. A synthetic muscle-specific promoter comprising a liver de-targeting element.
13. The synthetic muscle-specific promoter according to clause 12 wherein the liver de-targeting element is selected from the group consisting of:
   - SEQ ID NO: 15 or a functional variant thereof;
   - SEQ ID NO: 13 or a functional variant thereof;
   - SEQ ID NO: 17 or a functional variant thereof; or
   - SEQ ID NO: 14 or a functional variant thereof.
14. The synthetic muscle-specific promoter according to clause 12 or 13 wherein the liver de-targeting element comprises or consists of a sequence which is at least 70%, 75%, 80%, 85%, 90%, 95%, 96%, 97%, 98% or 99% identical to SEQ ID NO: 15, SEQ ID NO: 13, SEQ ID NO: 17 or SEQ ID NO: 14.
15. An expression cassette comprising a synthetic muscle-specific promoter according to any one of clauses 6-14 operably linked to a sequence encoding an expression product or an expression cassette comprising a synthetic muscle-specific promoter comprising CRE0145 or a functional variant thereof and DES_MT_enhancer_48bp or a functional variant thereof, wherein the synthetic muscle-specific promoter is operably linked to an expression product and a target sequence for miR122.
16. A vector comprising a synthetic muscle-specific promoter according to any one of clauses 6-14 or an expression cassette according to clause 15.
17. The vector of clause 16 which is an AAV vector, an adenoviral vector, a retroviral vector or a lentiviral vector.
18. A gene therapy vector comprising a synthetic promoter comprising a liver de-targeting element is selected from the group consisting of:
   - SEQ ID NO: 15 or a functional variant thereof;
   - SEQ ID NO: 13 or a functional variant thereof;
   - SEQ ID NO: 17 or a functional variant thereof; or
   - SEQ ID NO: 14 or a functional variant thereof.
19. The gene therapy vector according to clause 18 comprising or consisting of a sequence which is at least 70%, 75%, 80%, 85%, 90%, 95%, 96%, 97%, 98% or 99% identical to SEQ ID NO: 15, SEQ ID NO: 17, SEQ ID NO: 13 or SEQ ID NO: 14.
20. The gene therapy vector according to clause 18 or 19 wherein the gene therapy vector is an AAV vector.
21. A gene therapy AAV vector comprising an expression cassette, the expression cassette comprising a synthetic promoter operably linked to a sequence encoding an expression product and a target sequence for miR122.
22. The gene therapy AAV vector according to clause 21 wherein the target sequence for miR122 comprises or consists of SEQ ID NO: 16 or SEQ ID NO: 19 or a functional variant thereof.
23. The gene therapy AAV vector according to clause 21 or 22, wherein the target sequence for miR122 comprises or consist of a sequence which is at least 70%, 75%, 80%, 85%, 90%, 95%, 96%, 97%, 98% or 99% identical to SEQ ID NO: 16 or SEQ ID NO: 19.
24. A virion comprising a vector according to any one of clauses 16-23.
25. A pharmaceutical composition comprising a synthetic muscle-specific promoter according to any one of clauses 6-14, expression cassette according to clause 15, a vector according to any one of clauses 16-23 or a virion according to clause 24.
26. A synthetic muscle-specific promoter according to any one of clauses 6-14, expression cassette according to clause 15, a vector according to any one of clauses 16-23, a virion according to clause 24, or a pharmaceutical composition according to clause 25 for use in therapy.
27. A cell comprising a synthetic muscle-specific promoter according to any one of clauses 6-14, expression cassette according to clause 15, a vector according to any one of clauses 16-23 or a virion according to clause 24.
28. A synthetic muscle-specific promoter according to any one of clauses 6-14, expression cassette according to clause 15, a vector according to any one of clauses 16-23, a virion according to clause 24, or a pharmaceutical composition according to clause 25 for use in the manufacture of a pharmaceutical composition for the treatment of a medical condition or disease.
29. A method for producing an expression product, the method comprising providing an expression cassette according to clause 15 in a muscle cell and expressing the expression product present in the expression cassette.
30. A method for expressing a therapeutic transgene in a muscle cell, the method comprising introducing into the muscle cell an expression cassette according to clause 15, a vector according to any one of clauses 16-23 or a virion according to clause 24.
31. A method of therapy of a subject, preferably a human, in need thereof, the method comprising:
   administering to the subject an expression cassette according to clause 15, a vector according to any one of clauses 16-23, a virion according to clause 24 or a pharmaceutical composition according to clause 25, which comprises a sequence encoding a therapeutic product operably linked to a promoter according any one of clauses 6-14; and
   expressing a therapeutic amount of the therapeutic product in the muscle of said subject.
32. The method of therapy of a subject of clause 31, wherein the therapeutic amount of the therapeutic product is expressed in the skeletal and/or cardiac muscle.

The sequence listing is filed as part of the description and is submitted to the Greek Patent Office in electronic format.

## Claims

1. A synthetic muscle-specific cis-regulatory module (CRM) comprising CRE0145 or a functional variant thereof, DES_MT enhancer 48bp or a functional variant thereof and at least one de-targeting element

2. The synthetic muscle-specific CRM according to claim 1 wherein the at least one de-targeting element is a liver de-targeting element and is selected from the group consisting of:
- Liver de-targeting sequence 1 (SEQ ID NO: 13) or a functional variant thereof;
- Liver de-targeting sequence 2 (SEQ ID NO: 14) or a functional variant thereof;
- Liver de-targeting sequence 3 (SEQ ID NO: 17) or a functional variant thereof;
- ZBTB20 binding site (SEQ ID NO: 15) or a functional variant thereof;
- Mir122 miRNA target sequence 1 (SEQ ID NO: 16) or a functional variant thereof; and
- Mir122 miRNA target sequence 2 (SEQ ID NO: 19) or a functional variant thereof.

3. The synthetic muscle-specific CRM according to any preceding claim wherein the CRM comprises a combination of regulatory elements, or functional variants thereof, selected from the group consisting of:
- CRE0145, DES_MT_enhancer_48bp and Liver de-targeting sequence 1;
- CRE0145, DES_MT enhancer 48bp and Liver de-targeting sequence 2;
- CRE0145, DES_MT enhancer 48bp and Liver de-targeting sequence 3;
- CRE0145, DES_MT_enhancer_48bp and ZBTB20 binding site;
- CRE0145, Liver de-targeting sequence 3, DES_MT_enhancer_48bp and Liver de-targeting sequence 3; and
- CRE0145, ZBTB20 binding site, DES_MT_enhancer_48bp and ZBTB20 binding site, optionally wherein the regulatory elements are present in the CRM in the recited order and adjacent to each other.

4. The synthetic muscle-specific CRM according to any preceding claims comprising or consisting of SEQ ID NO: 20, 21, 22 and 9 or a functional variant thereof, optionally comprising or consisting of a sequence which is at least 70%, 75%, 80%, 85%, 90%, 95%, 96%, 97%, 98% or 99% identical to SEQ ID NO: 20, 21, 22 and 9.

5. A synthetic muscle-specific promoter comprising a CRM according to any one of claims 1-4 operably linked to a promoter element, optionally wherein the promoter element is SCP1 or a functional variant or CRE0053 (SEQ ID NO: 26) or a functional variant thereof, optionally wherein the synthetic muscle-specific promoter comprises or consists of SP0525 (SEQ ID NO: 1), SP0526 (SEQ ID NO: 2), SP0527 (SEQ ID NO: 3) or SP0528 (SEQ ID NO: 4) or a functional variant of any thereof, optionally wherein the synthetic muscle-specific promoter comprises or consists of a sequence which is at least 70%, 75%, 80%, 85%, 90%, 95%, 96%, 97%, 98% or 99% identical to any one of SEQ ID NO 1-4.

6. A synthetic muscle-specific promoter comprising a liver de-targeting element, optionally wherein the liver de-targeting element is selected from the group consisting of:
- SEQ ID NO: 15 or a functional variant thereof;
- SEQ ID NO: 13 or a functional variant thereof;
- SEQ ID NO: 17 or a functional variant thereof; or
- SEQ ID NO: 14 or a functional variant thereof, optionally wherein the liver de-targeting element comprises or consists of a sequence which is at least 70%, 75%, 80%, 85%, 90%, 95%, 96%, 97%, 98% or 99% identical to SEQ ID NO: 15, SEQ ID NO: 13 or SEQ ID NO: 14.

7. An expression cassette comprising a synthetic muscle-specific promoter according to any one of claims 5-6 operably linked to a sequence encoding an expression product or an expression cassette comprising a synthetic muscle-specific promoter comprising CRE0145 or a functional variant thereof and DES_MT_enhancer_48bp or a functional variant thereof operably linked to an expression product and a target sequence for miR122.

8. A vector comprising a synthetic muscle-specific promoter according to any one of claims 5-6 or an expression cassette according to claim 7, optionally wherein the vector is an AAV vector, an adenoviral vector, a retroviral vector or a lentiviral vector.

9. A gene therapy AAV vector comprising an expression cassette, the expression cassette comprising a synthetic promoter operably linked to a sequence encoding an expression product and a target sequence for miR122, optionally wherein the target sequence for miR122 comprises or consist of SEQ ID NO: 16 or SEQ ID NO: 19 or a functional variant thereof, optionally wherein the target sequence for miR122 comprises or consist of a sequence which is at least 70%, 75%, 80%, 85%, 90%, 95%, 96%, 97%, 98% or 99% identical to SEQ ID NO: 16 or SEQ ID NO: 19.

10. A virion comprising a vector according to any one of claims 8-9.

11. A pharmaceutical composition comprising a synthetic muscle-specific promoter according to any one of claims 5-6, expression cassette according to claim 7, a vector according to any one of claims 8-9 or a virion according to claim 10.

12. A synthetic muscle-specific promoter according to any one of claims 5-6, expression cassette according to claim 7, a vector according to any one of claims 8-9, a virion according to claim 10, or a pharmaceutical composition according to claim 11 for use in therapy.

13. A cell comprising a synthetic muscle-specific promoter according to any one of claims 5-6, expression cassette according to claim 7, a vector according to any one of claims 8-9 or a virion according to claim 10.

14. A synthetic muscle-specific promoter according to any one of claims 5-6, expression cassette according to claim 7, a vector according to any one of claims 8-9, a virion according to claim 10, or a pharmaceutical composition according to claim 11 for use in the manufacture of a pharmaceutical composition for the treatment of a medical condition or disease.

15. A method for producing an expression product *ex vivo,* the method comprising providing an expression cassette according to claim 7 in a muscle cell and expressing the expression product present in the expression cassette.
